(19) Europäisches Patentamt
European Patent Office
Office européen des brevets

(11) EP 2 826 079 B1

(12) **EUROPÄISCHE PATENTSCHRIFT**

(45) Veröffentlichungstag und Bekanntmachung des Hinweises auf die Patenterteilung:
**20.01.2021 Patentblatt 2021/03**

(21) Anmeldenummer: **13707555.2**

(22) Anmeldetag: **05.03.2013**

(51) Int Cl.:
***H01L 51/52*** (2006.01) ***H01L 51/54*** (2006.01)
***C07C 211/61*** (2006.01)

(86) Internationale Anmeldenummer:
**PCT/EP2013/000641**

(87) Internationale Veröffentlichungsnummer:
**WO 2013/135352 (19.09.2013 Gazette 2013/38)**

(54) **ELEKTRONISCHE VORRICHTUNGEN**
ELECTRONIC DEVICES
DISPOSITIFS ÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.03.2012 EP 12001759**

(43) Veröffentlichungstag der Anmeldung:
**21.01.2015 Patentblatt 2015/04**

(60) Teilanmeldung:
**18205568.1 / 3 460 864**

(73) Patentinhaber: **Merck Patent GmbH**
**64293 Darmstadt (DE)**

(72) Erfinder:
- **PFLUMM, Christof**
**64291 Darmstadt (DE)**
- **VOGES, Frank**
**67098 Bad Duerkheim (DE)**
- **KROEBER, Jonas Valentin**
**60311 Frankfurt am Main (DE)**
- **STOESSEL, Philipp**
**60487 Frankfurt am Main (DE)**
- **KAISER, Joachim**
**64289 Darmstadt (DE)**

(56) Entgegenhaltungen:
**EP-A1- 1 623 970**
**EP-A1- 1 713 136**
**EP-A2- 2 343 277**
**EP-A2- 2 365 555**
**WO-A1-2006/015567**
**WO-A1-2007/072952**
**JP-A- H09 281 728**
**JP-A- 2007 284 431**
**US-A1- 2007 082 226**
**US-A1- 2009 206 744**
**US-A1- 2009 284 140**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 826 079 B1

## Beschreibung

Die vorliegende Anmeldung betrifft eine elektronische Vorrichtung enthaltend Anode, Kathode, mindestens eine emittierende Schicht zwischen Anode und Kathode, mindestens eine p-dotierte Schicht A, welche ein Mono-Triarylamin als Host enthält, mindestens eine nicht p-dotierte Schicht B enthaltend ein Mono-Triarylamin, sowie eine weitere nicht p-dotierte Schicht C enthaltend ein Mono-Triarylamin. Insbesondere betrifft die Erfindung eine organische Elektrolumineszenzvorrichtung (OLED) enthaltend den oben genannten Schichtaufbau.

Elektronische Vorrichtungen enthaltend organische Schichten sind aktuell Gegenstand intensiver Forschung. Von besonderem Interesse sind organische Elektrolumineszenzvorrichtungen, die beispielsweise in Displays von mobilen elektronischen Geräten oder als Beleuchtungselemente verwendet werden.

Im Stand der Technik bekannt, z. B. in EP 1463130 A2 und DE 102007031220 A1, sind elektronische Vorrichtungen enthaltend eine oder mehrere p-dotierte Schichten enthaltend einen Dotanden und ein Hostmaterial, wobei das Hostmaterial eine organische Stickstoffverbindung darstellen kann.

Weiterhin ist bekannt, beispielsweise aus US 5093698 A, dass durch Dotierung von Loch- oder Elektronentransportschichten in elektronischen Vorrichtungen deren Leitfähigkeit deutlich erhöht werden kann.

Weiterhin im Stand der Technik bekannt sind elektronische Vorrichtungen enthaltend Mono-Triarylamine als Materialien für die lochtransportierende oder emittierende Schicht, beispielsweise aus EP 1885008 A1 und JP 1995053955 A. Ebenso sind elektronische Vorrichtungen bestehend aus einer Emissionsschicht und einer Lochtransportschicht (HTL) zwischen 2 Elektroden bekannt, wobei die Lochtransportschicht aus einer undotierten HTL, einer p-dotierten HTL und einer undotierten HTL bestehen kann, wie in EP 2 365 555 A2 offenbart, wobei eine undotierte HTL an die Anode angrenzt.

Äußerst wichtig bei der Entwicklung neuer elektronischer Vorrichtungen sind die Langzeitstabilität der Vorrichtungen und ihre Leistungsdaten, insbesondere Betriebsspannung und Effizienz. Obwohl Fortschritte erzielt worden sind, besteht in allen diesen Punkten weiterhin Verbesserungsbedarf gegenüber den im Stand der Technik bekannten Ausführungsformen.

Technische Aufgabe der vorliegenden Erfindung ist die Bereitstellung einer elektronischen Vorrichtung, welche in einem oder mehreren der oben genannten Punkte Langzeitstabilität und Leistungsdaten bessere Eigenschaften aufweist als die im Stand der Technik bekannten Vorrichtungen.

Die technische Aufgabe wird durch die im Folgenden aufgeführten Gegenstände der Erfindung gelöst.

Eine elektronische Vorrichtung, enthaltend

- Anode
- Kathode,
- mindestens eine emittierende Schicht, welche zwischen Anode und Kathode angeordnet ist und je eine oder mehrere Emitterverbindungen enthält, die phosphoreszierend oder fluoreszierend sein können,
- mindestens eine p-dotierte Schicht A, welche zwischen Anode und emittierender Schicht angeordnet ist, und die ein Mono-Triarylamin als Host enthält, sowie
- mindestens eine nicht p-dotierte Schicht B, welche zwischen Anode und emittierender Schicht angeordnet ist, enthaltend ein Mono-Triarylamin, und
- mindestens eine nicht p-dotierte Schicht C, enthaltend ein Mono-Triarylamin, zwischen der Schicht B und der emittierenden Schicht, dadurch gekennzeichnet, dass die p-dotierte Schicht A direkt angrenzend an die Anode angeorndet ist, und dass die Schicht C unmittelbar an die emittierende Schicht angrenzt.

Dabei wird unter einer p-dotierten Schicht eine Schicht verstanden, in der freie Löcher erzeugt wurden und deren Leitfähigkeit dadurch erhöht ist. Eine umfassende Diskussion von dotierten Transportschichten in OLEDs oder in organischen Solarzellen findet sich in Chem. Rev. 2007, 107, 1233.

Unter einem Mono-Triarylamin wird eine Verbindung verstanden, welche eine einzige Triarylamingruppe aufweist. Eine Triarylamingruppe ist eine Gruppe, in der drei Aryl- oder Heteroarylgruppen an ein Stickstoffatom gebunden sind. Bevorzugt enthält das Mono-Triarylamin keine weitere Arylaminogruppe. Besonders bevorzugt enthält das Mono-Triarylamin keine weitere Aminogruppe.

Die elektronische Vorrichtung weist einen Schichtaufbau auf, bei dem Schicht A und Schicht B zwischen Anode und emittierender Schicht angeordnet sind, wobei die Schicht A direkt an die Anode angrenzend anodenseitig von Schicht B angeordnet ist.

Weiterhin bevorzugt stellen Schicht A, Schicht B und die emittierende Schicht organische Schichten dar, d.h. Schichten, welche im Wesentlichen aus einer oder mehreren organischen Verbindungen bestehen. Die erfinderische Vorrichtung weist die folgenden Schichtfolge auf, wobei die Schicht A direkt an die Anode und die Schicht C unmittelbar an die emittierenden Schicht angrenzt.

Bevorzugt ist weiterhin eine Vorrichtung, die die folgende Schichtenfolge aufweist zwischen Anode und emittierender Schicht, wobei die Schichten unmittelbar aneinander angrenzen:

- Anode
- Schicht A
- Schicht B
- Schicht C
- emittierende Schicht.

Diese Ausführungsform entspricht der in Fig. 3 abgebildeten Struktur der elektronischen Vorrichtung (1), wobei Anode (2), Schicht A (3), Schicht B (4), Schicht C (4a), emittierende Schicht (5), Elektronentransportschicht (6) und Kathode (7) aufeinander abfolgen und unmittelbar aneinander angrenzen.

Bevorzugt ist weiterhin eine Elektroneninjektionsschicht (6a) zwischen Elektronentransportschicht (6) und Kathode (7) vorhanden, wie in Fig. 2 gezeigt.

Allgemein kann die elektronische Vorrichtung auch weitere Schichten enthalten, beispielsweise zusätzliche Lochtransportschichten, Elektronentransportschichten, Auskopplungsschichten, Zwischenschichten (Interlayers), Lochblockierschichten, Elektronenblockierschichten, Excitonenblockierschichten, Ladungserzeugungsschichten und/oder organische oder anorganische p/n-Übergänge. Dem Fachmann ist die bevorzugte Anordnung und Funktion und Kombination solcher Schichten bekannt.

Die Schichten A, B und C zwischen Anode und emittierender Schicht und die emittierende Schicht enthalten alle mindestens ein Mono-Triarylamin. Besonders bevorzugt enthalten sie keine anderen Triarylamin-Verbindungen außer Mono-Triarylaminen. Ganz besonders bevorzugt sind ein oder mehrere Mono-Triarylamine identisch in allen Schichten zwischen Anode und emittierender Schicht vorhanden.

Die p-dotierte Schicht A hat bevorzugt eine Stärke von 1 bis 500 nm, besonders bevorzugt von 5 bis 300 nm und ganz besonders bevorzugt von 8 bis 250 nm

Die p-dotierte Schicht A enthält bevorzugt einen Dotanden, der eine Elektronenakzeptorverbindung ist. Besonders bevorzugt ist der Dotand in der Lage, das Mono-Triarylamin zu oxidieren, hat also ein ausreichend hohes Redoxpotential, insbesondere ein höheres Redoxpotential als das Mono-Triarylamin.

Gemäß einer bevorzugten Ausführungsform hat der Dotand ein LUMO, welches nicht nicht höher liegt als 0.3 eV über dem HOMO des Mono-Triarylamins, bevorzugt nicht höher als 0.2 eV und besonders bevorzugt nicht höher als 0.1 eV. Am stärksten bevorzugt hat der Dotand ein LUMO, welches gleich oder niedriger liegt als das HOMO des Mono-Triarylamins.

HOMO- und LUMO-Lagen werden im Rahmen der vorliegenden Anmeldung durch quantenchemische Rechnungen mit "Gaussian03W" (Gaussian Inc.) bestimmt, wie in den Ausführungsbeispielen explizit angegeben wird.

Bevorzugt hat der Dotand ein LUMO, welches kleiner als -4.6 eV ist, besonders bevorzugt kleiner als -4.8 eV und ganz besonders bevorzugt kleiner als -5.0 eV ist. Am stärksten bevorzugt hat der Dotand ein LUMO, das kleiner als -5.1 eV ist.

Als Dotanden sind prinzipiell alle Verbindungen geeignet, welche Elektronenakzeptorverbindungen darstellen und die Leitfähigkeit der organischen Schicht durch Oxidation des Hosts erhöhen können. Der Fachmann kann im Rahmen seines allgemeinen Fachwissens ohne größeren Aufwand geeignete Verbindungen identifizieren.

Insbesondere geeignet als Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709, US 2010/0096600 und WO 2012/095143 offenbarten Verbindungen.

Der Dotand der p-dotierten Schicht A ist bevorzugt gewählt aus Chinodimethanverbindungen, Azaindenofluorendionen, Azaphenalenen, Azatriphenylenen, $I_2$, Metallhalogeniden, bevorzugt Übergangsmetallhalogeniden, Metalloxiden, bevorzugt Metalloxiden enthaltend mindestens ein Übergangsmetall oder ein Metall der 3. Hauptgruppe, und Übergangsmetallkomplexen, bevorzugt Komplexen von Cu, Co, Ni, Pd und Pt mit Liganden enthaltend mindestens ein Sauerstoffatom als Bindungsstelle.

Bevorzugt sind weiterhin Übergangsmetalloxide als Dotanden, bevorzugt Oxide von Rhenium, Molybdän und Wolfram, besonders bevorzugt $Re_2O_7$, $MoO_3$, $WO_3$ und $ReO_3$.

Bevorzugt sind weiterhin organische Verbindungen mit Elektronenakzeptoreigenschaften als Dotanden, insbesondere die folgenden Verbindungen:

| | | |
|---|---|---|
| 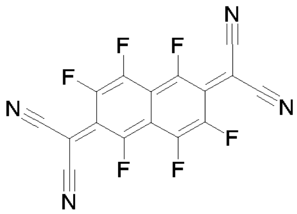 | 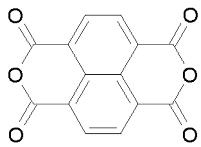 | 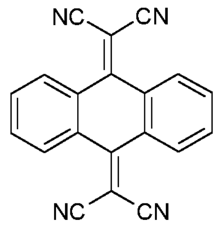 |
| (D-1) | (D-2) | (D-3) |
| 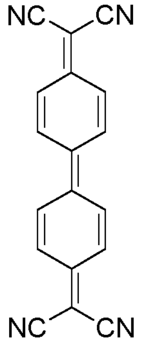 | 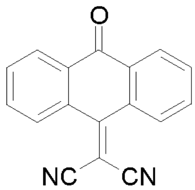 | 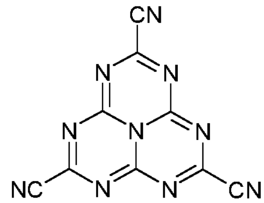 |
| (D-4) | (D-5) | (D-6) |
| 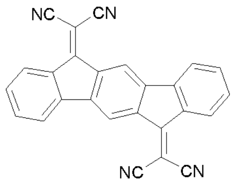 | 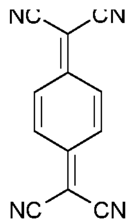 | 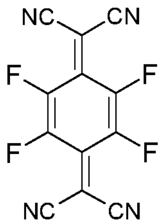 |
| (D-7) | (D-8) | (D-9) |
| 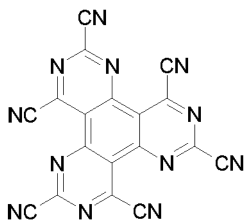 | 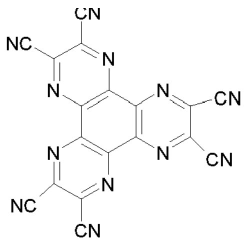 | 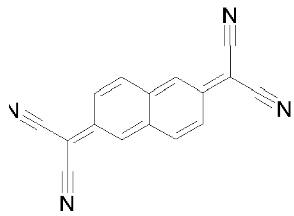 |
| (D-10) | (D-11) | (D-12) |

Bevorzugt liegt der Dotand in der Schicht A in einer Konzentration von 0.1 bis 20 Vol-%, bevorzugt 0.5 bis 12 Vol-%, besonders bevorzugt 1 bis 8 Vol-% und ganz besonders bevorzugt 2 bis 6 Vol-% vor.

Die p-dotierte Schicht A kann zusätzlich zum Dotanden und dem Mono-Triarylamin noch weitere Verbindungen enthalten, beispielsweise weitere Dotanden und/oder weitere Verbindungen mit lochtransportierenden Eigenschaften.

Die p-dotierte Schicht hat gemäß einer bevorzugten Ausführungsform eine Leitfähigkeit von mehr als $10^{-8}$ S/cm, besonders bevorzugt mehr als $10^{-7}$ S/cm, ganz besonders bevorzugt mehr als $10^{-6}$ S/cm und am stärksten bevorzugt mehr als $10^{-5}$ S/cm.

Leitfähigkeiten von dünnen Schichten können mit der Zweipunktmethode gemessen werden. Dabei werden auf ein Substrat Kontakte aus einem leitfähigen Material aufgebracht, zum Beispiel Gold oder Indium-Zinn-Oxid. Danach wird die zu untersuchende dünne Schicht großflächig auf das Substrat aufgebracht, so dass die Kontakte von der Schicht überdeckt werden. Nach Anlegen einer Spannung an die Kontakte wird der dann fließende Strom gemessen. Aus der Geometrie der Kontakte und der Schichtdicke der Probe ergibt sich aus dem so bestimmten Widerstand die Leitfähigkeit der Schicht. Solche Messungen an dotierten organischen Schichten sind beispielsweise in EP 1786050 A1 beschrieben. Alternativ kann die Vierpunkt-Methode zur Bestimmung der Leitfähigkeit eingesetzt werden, wie in van der Pauw et al.,

Philips Technical Review, 1959/1960, Vol. 20, 220 und van der Pauw et al., Philips Research Reports 1958, Vol. 13, 1, beschrieben.

Gemäß einer bevorzugten Ausführungsform ist dasselbe Mono-Triarylamin in Schicht A und in Schicht B vorhanden.

Gemäß einer weiteren bevorzugten Ausführungsform ist dasselbe Mono-Triarylamin in Schicht B und in Schicht C vorhanden.

Gemäß einer weiteren bevorzugten Ausführungsform ist dasselbe Mono-Triarylamin in Schicht A und in Schicht C vorhanden.

Gemäß einer weiteren bevorzugten Ausführungsform ist dasselbe Mono-Triarylamin in Schicht A, in Schicht B und in Schicht C vorhanden.

Es ist bevorzugt, dass das Mono-Triarylamin, insbesondere das Mono-Triarylamin der Schicht A und B, eine Verbindung der Formel (I) ist

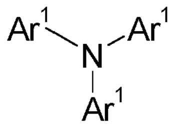

Formel (I)

wobei gilt:

$Ar^1$ ist bei jedem Auftreten gleich oder verschieden ein aromatisches oder heteroaromatisches Ringsystem mit 5 bis 60 aromatischen Ringatomen, welches durch einen oder mehrere Reste $R^1$ substituiert sein kann;

$R^1$ ist bei jedem Auftreten gleich oder verschieden H, D, F, Cl, Br, I, CHO, $C(=O)R^2$, $P(=O)(R^2)_2$, $S(=O)R^2$, $S(=O)_2R^2$, $CR^2=CR^2R^2$, CN, $NO_2$, $Si(R^2)_3$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden; und

$R^2$ ist bei jedem Auftreten gleich oder verschieden H, D, CN oder ein aliphatischer, aromatischer und/oder heteroaromatischer Kohlenwasserstoffrest mit 1 bis 20 C-Atomen, in dem auch H-Atome durch D oder F ersetzt sein können; dabei können zwei oder mehrere benachbarte Substituenten $R^2$ auch miteinander ein mono- oder polycyclisches, aliphatisches oder aromatisches Ringsystem bilden.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein $sp^3$-hybridisiertes C-, Si-, N- oder O-Atom, ein $sp^2$-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches

noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Bevorzugt ist $Ar^1$ ein aromatisches oder heteroaromatisches Ringsystem mit 6 bis 40 aromatischen Ringatomen, besonders bevorzugt ein aromatisches Ringsystem mit 6 bis 30 aromatischen Ringatomen, ganz besonders bevorzugt ein aromatisches Ringsystem mit 12 bis 30 aromatischen Ringatomen.

Es ist bevorzugt, dass mindestens eine der Gruppen $Ar^1$ ein aromatisches Ringsystem mit 12 bis 30 aromatischen Ringatomen darstellt, und bevorzugt gewählt ist aus Biphenyl, Terphenyl, Quaterphenyl, Spirobifluoren, Fluoren und Indenofluoren, welches jeweils durch einen oder mehrere Reste $R^1$ substituiert sein kann.

Allgemein ist $R^1$ bevorzugt bei jedem Auftreten gleich oder verschieden H, D, F, CN, $Si(R^2)_3$, $N(R^2)_2$, eine geradkettige Alkyl- oder Alkoxygruppe mit 1 bis 20 C-Atomen oder eine verzweigte oder cyclische Alkyl- oder Alkoxygruppe mit 3 bis 20 C-Atomen, wobei die oben genannten Gruppen jeweils mit einem oder mehreren Resten $R^2$ substituiert sein können und wobei in den oben genannten Gruppen eine oder mehrere $CH_2$-Gruppen durch -C=C-, $-R^2C=CR^2-$, $Si(R^2)_2$, C=O, $C=NR^2$, $-NR^2-$, -O-, -S-, -C(=O)O- oder $-C(=O)NR^2-$ ersetzt sein können, oder ein aromatisches oder heteroaromatisches Ringsystem mit 5 bis 20 aromatischen Ringatomen, das jeweils mit einem oder mehreren Resten $R^2$ substituiert sein kann, wobei zwei oder mehr Reste $R^1$ miteinander verknüpft sein können und einen Ring bilden können.

Bevorzugt entspricht das Mono-Triarylamin einer der Formeln (II), (III) oder (IV)

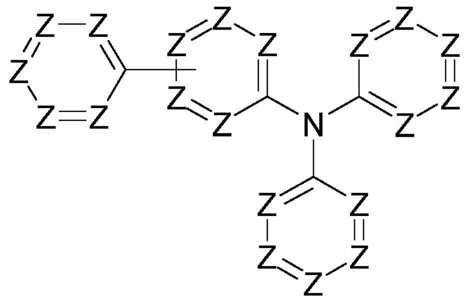

Formel (II)

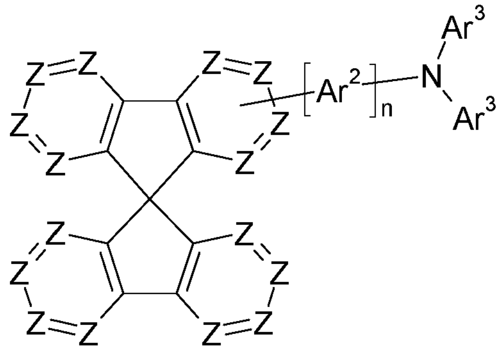

Formel (III)

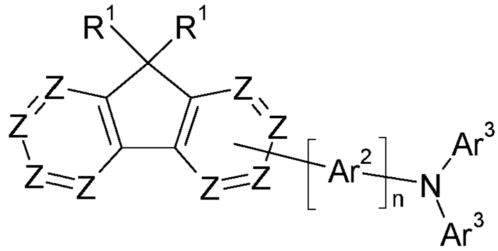

Formel (IV)

wobei gilt:

Z ist bei jedem Auftreten gleich oder verschieden N oder $CR^1$, wobei Z gleich C ist, wenn ein Substituent gebunden ist;

$Ar^2$ ist ein aromatisches oder heteroaromatisches Ringsystem mit 5 bis 20 aromatischen Ringatomen, welches durch einen oder mehrere Reste $R^1$ substituiert sein kann;

$Ar^3$ ist bei jedem Auftreten gleich oder verschieden ein aromatisches oder heteroaromatisches Ringsystem mit 5 bis 60 aromatischen Ringatomen, welches durch einen oder mehrere Reste $R^1$ substituiert sein kann;

$R^1$ ist bei jedem Auftreten gleich oder verschieden H, D, F, Cl, Br, I, CHO, $C(=O)R^2$, $P(=O)(R^2)_2$, $S(=O)R^2$, $S(=O)_2R^2$, $CR^2=CR^2R^2$, CN, $NO_2$, $Si(R^2)_3$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, C=C , $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder

CONR$^2$ ersetzt sein können und wobei ein oder mehrere H-Atome durch D, F, Cl, Br, I, CN oder $NO_2$ ersetzt sein können, oder ein aromatisches oder heteroaromatisches Ringsystem mit 5 bis 60 aromatischen Ringatomen, das jeweils durch einen oder mehrere Reste $R^2$ substituiert sein kann, oder eine Aryloxy- oder Heteroaryloxygruppe mit 5 bis 60 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;

$R^2$ ist bei jedem Auftreten gleich oder verschieden H, D, CN oder ein aliphatischer, aromatischer und/oder heteroaromatischer Kohlenwasserstoffrest mit 1 bis 20 C-Atomen, in dem auch H-Atome durch D oder F ersetzt sein können; dabei können zwei oder mehrere benachbarte Substituenten $R^2$ auch miteinander ein mono- oder polycyclisches, aliphatisches oder aromatisches Ringsystem bilden; und

n ist 0 oder 1.

Bevorzugt sind in den Formeln (II), (III) und (IV) nicht mehr als drei Gruppen Z pro aromatischem Ring gleich N. Besonders bevorzugt sind 0, 1 oder 2 Gruppen Z pro aromatischem Ring gleich N. Ganz besonders bevorzugt sind alle Gruppen Z gleich CR$^1$ oder, wenn ein Substituent gebunden ist, gleich C.

Bevorzugt ist $Ar^2$ in den Formeln (II), (III) und (IV) ein aromatisches oder heteroaromatisches Ringsystem mit 6 bis 18 aromatischen Ringatomen, besonders bevorzugt ein aromatisches Ringsystem mit 6 bis 18 aromatischen Ringatomen, das mit einem oder mehreren Resten $R^1$ substituiert sein kann.

Beispiele für Mono-Triarylamine zur Verwendung in den erfindungsgemäßen Vorrichtungen sind im Folgenden aufgeführt:

| | | |
|---|---|---|
| 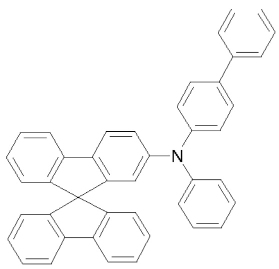 | 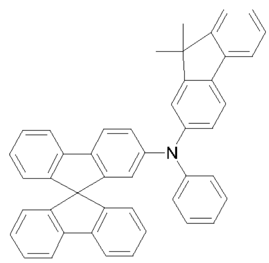 | 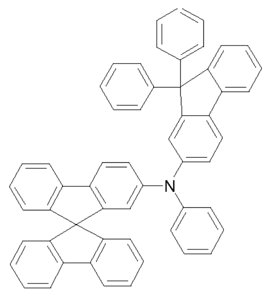 |
| (1) | (2) | (3) |
| 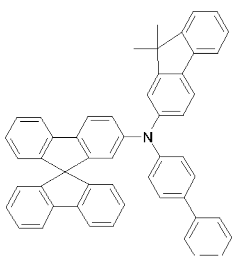 | 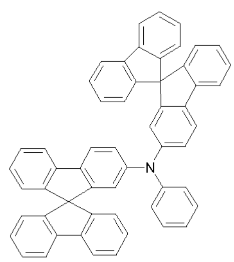 | 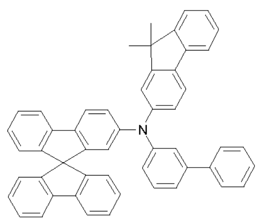 |
| (4) | (5) | (6) |
| 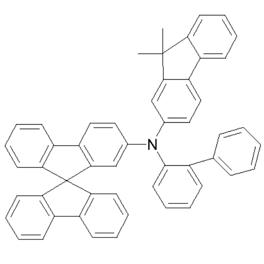 | 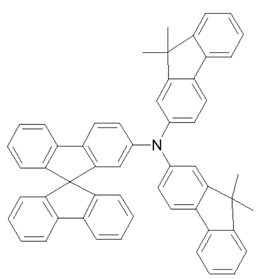 | 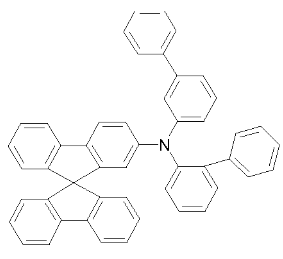 |
| (7) | (8) | (9) |

(fortgesetzt)

| | | |
|---|---|---|
| 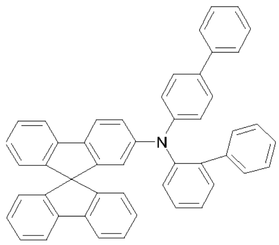 | 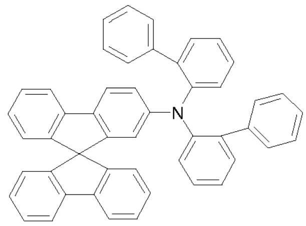 | 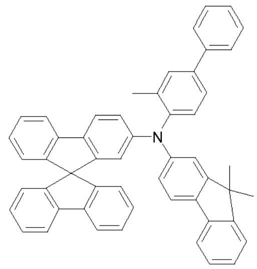 |
| (10) | (11) | (12) |
| 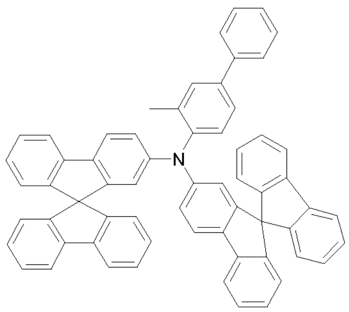 | 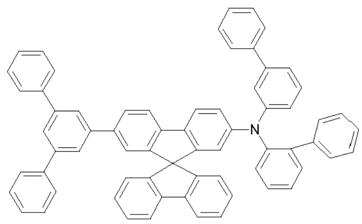 | 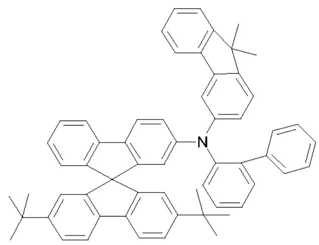 |
| (13) | (14) | (15) |
| 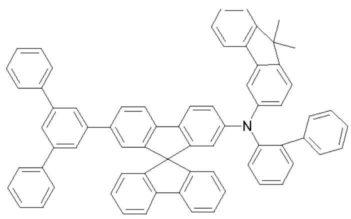 | 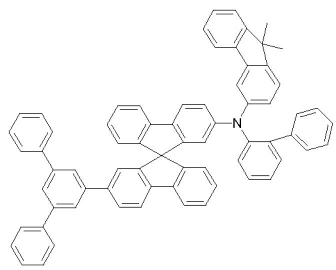 | 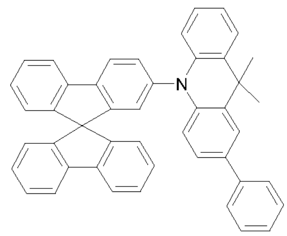 |
| (16) | (17) | (18) |
| 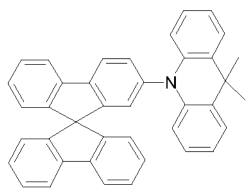 | 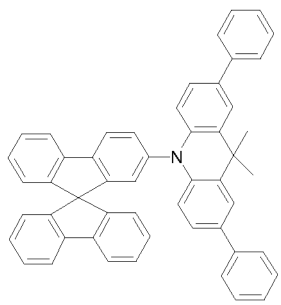 | 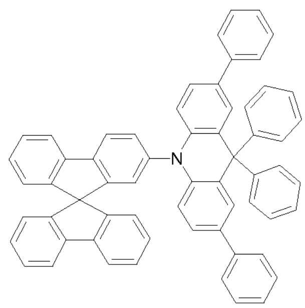 |
| (19) | (20) | (21) |
| 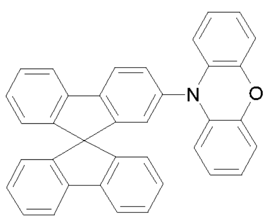 | 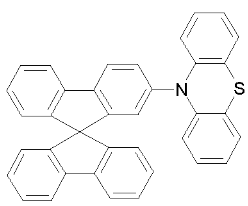 | 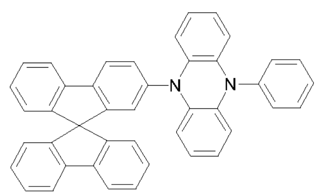 |
| (22) | (23) | (24) |

(fortgesetzt)

| | | |
|---|---|---|
| 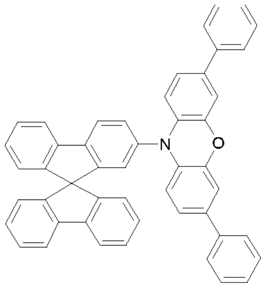 | 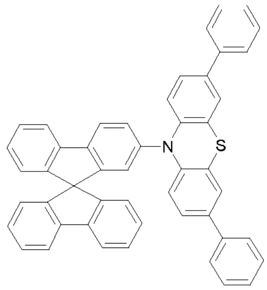 | 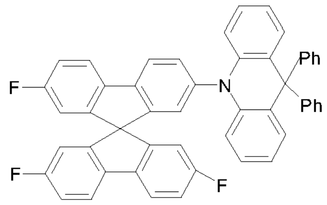 |
| (25) | (26) | (27) |
| 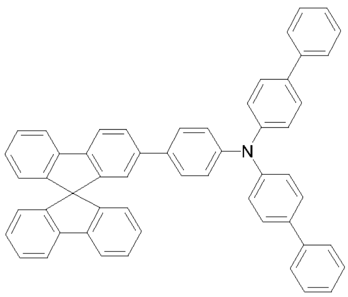 | 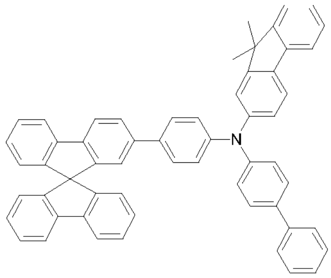 | 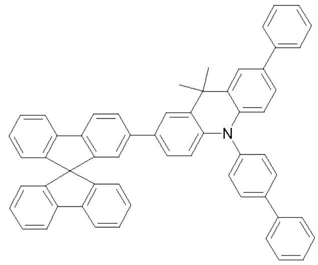 |
| (28) | (29) | (30) |
| 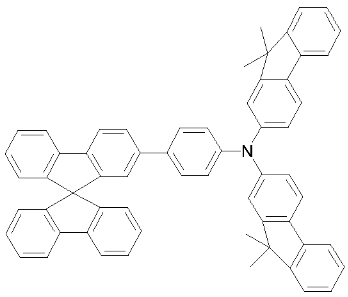 | 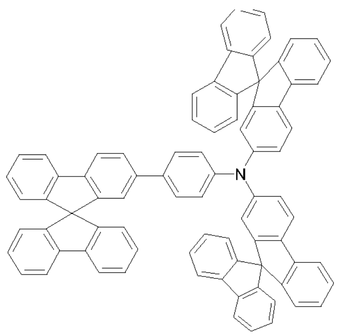 | 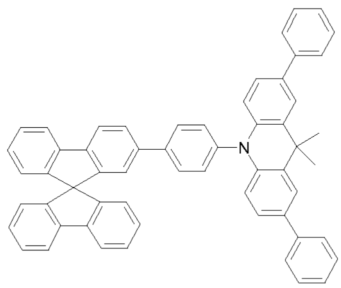 |
| (31) | (32) | (33) |
| 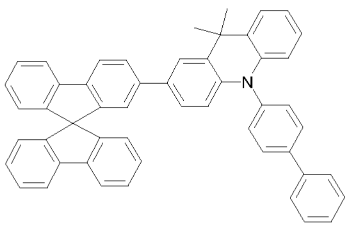 | 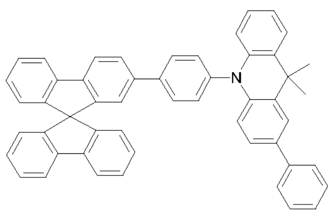 | 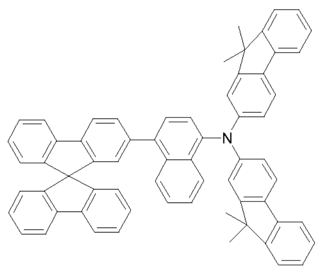 |
| (34) | (35) | (36) |
| 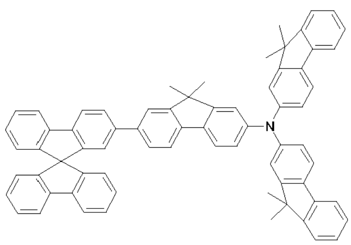 | 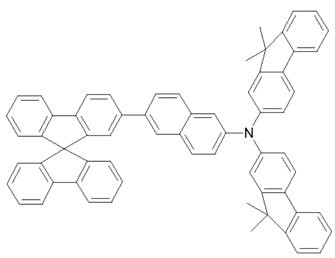 | 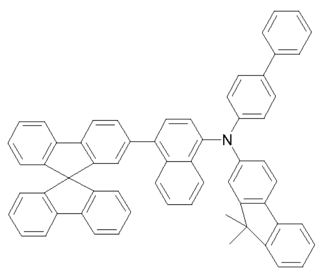 |

(fortgesetzt)

| (37) | (38) | (39) |
|---|---|---|
| 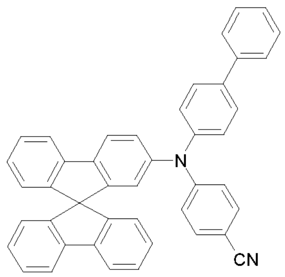 | 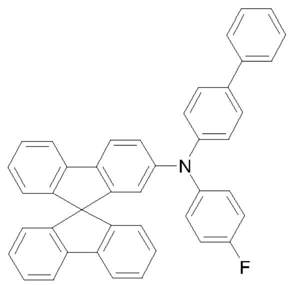 | 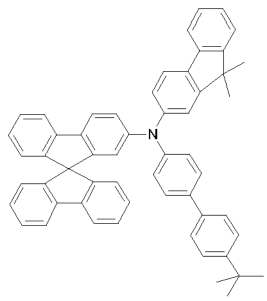 |
| (40) | (41) | (42) |
| 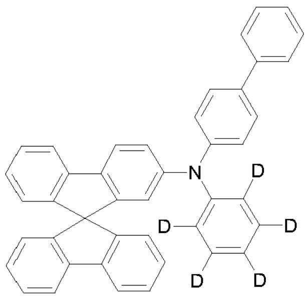 | 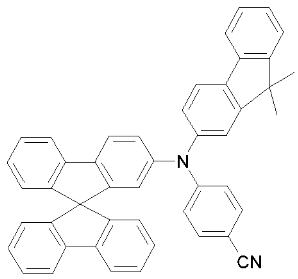 | 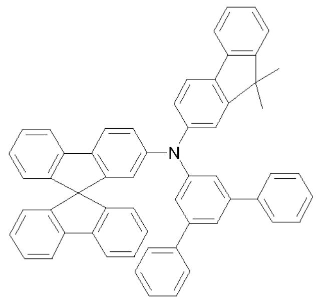 |
| (43) | (44) | (45) |
| 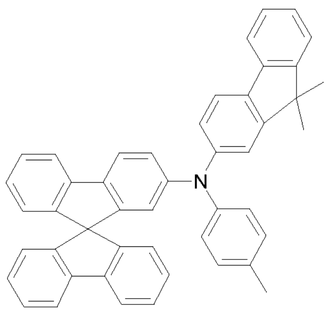 | 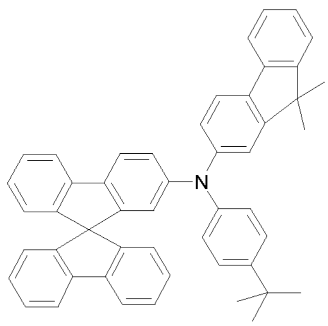 | 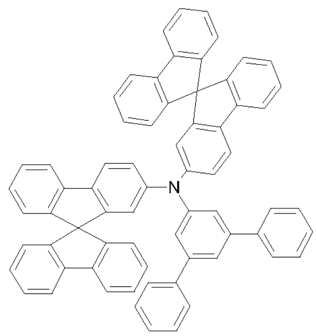 |
| (46) | (47) | (48) |
| 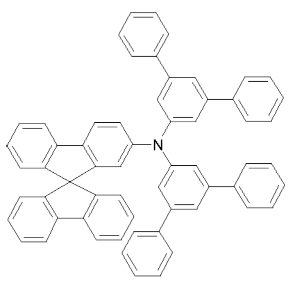 | 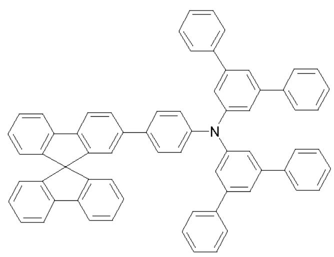 | 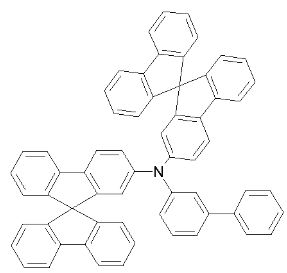 |
| (49) | (50) | (51) |

(fortgesetzt)

| | | |
|---|---|---|
| 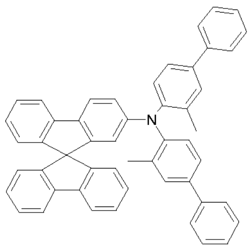 | 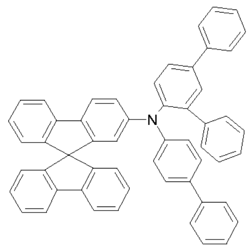 | 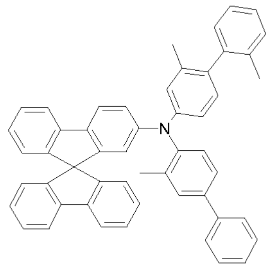 |
| (52) | (53) | (54) |
| 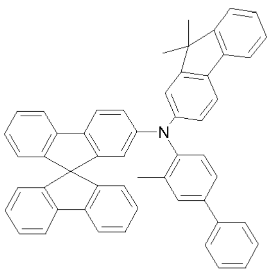 | 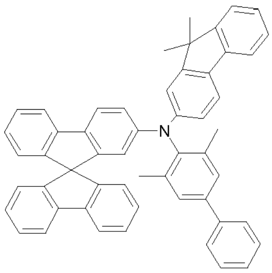 | 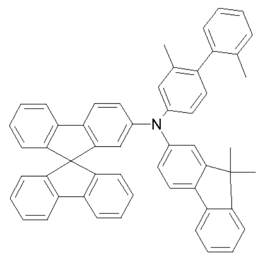 |
| (55) | (56) | (57) |
| 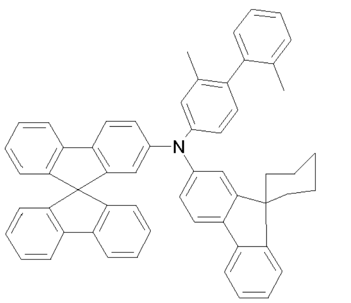 | 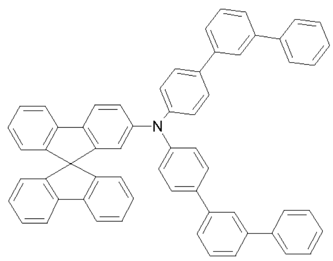 | 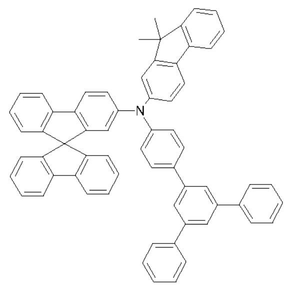 |
| (58) | (59) | (60) |
| 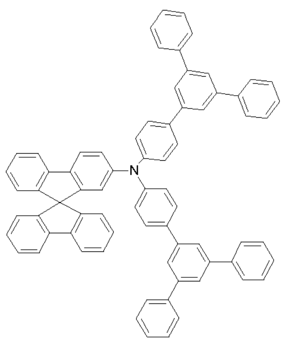 | 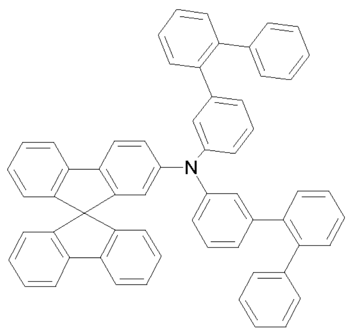 | 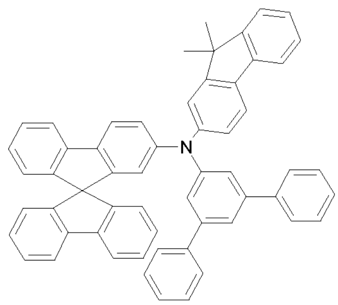 |
| (61) | (62) | (63) |
| 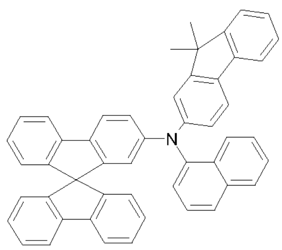 | 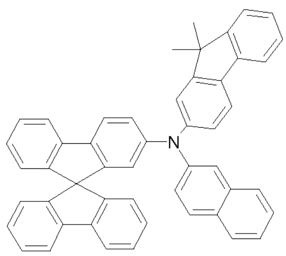 | 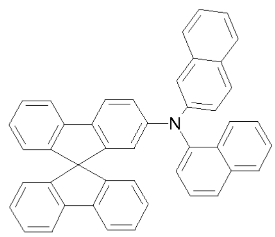 |

(fortgesetzt)

| (64) | (65) | (66) |
|---|---|---|
| 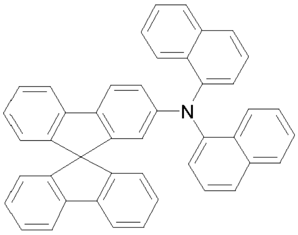 | 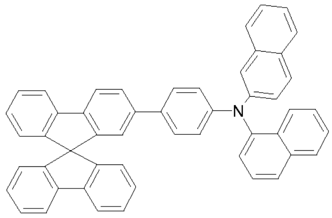 | 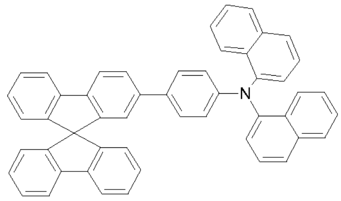 |
| (67) | (68) | (69) |
| 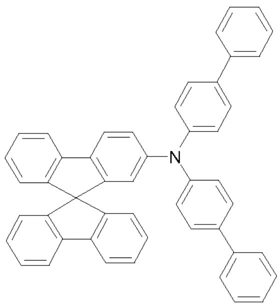 | 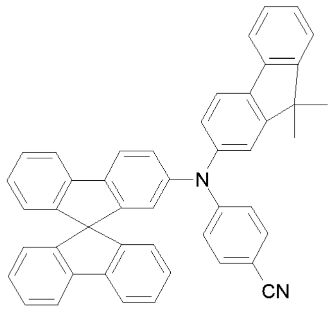 | 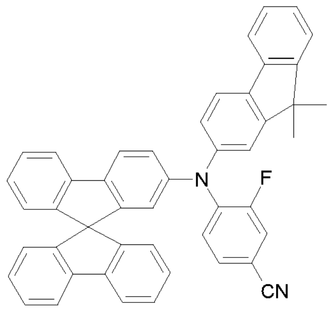 |
| (70) | (71) | (72) |
| 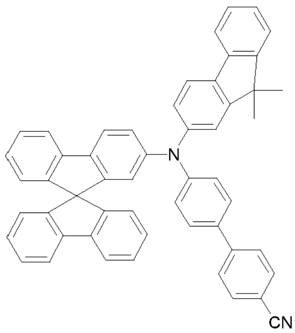 | 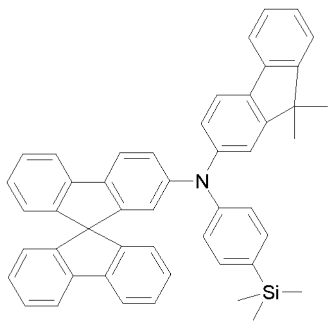 | 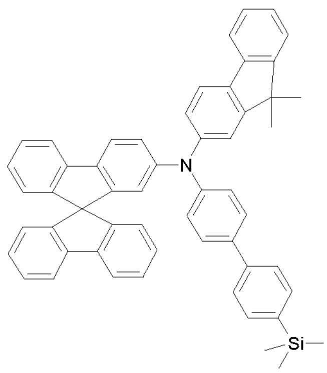 |
| (73) | (74) | (75) |
| 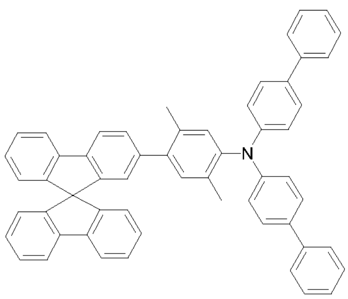 | 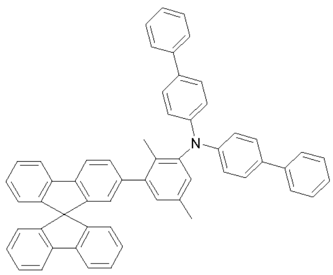 | 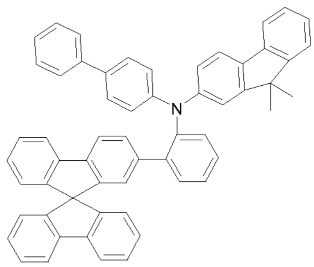 |
| (76) | (77) | (78) |

(fortgesetzt)

| | | |
|---|---|---|
| 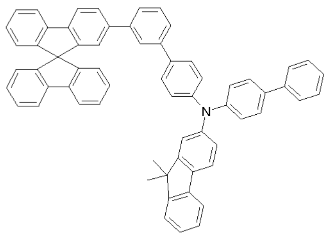 | 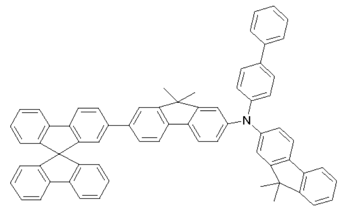 | 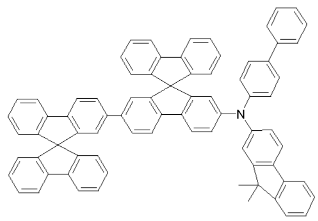 |
| (79) | (80) | (81) |
| 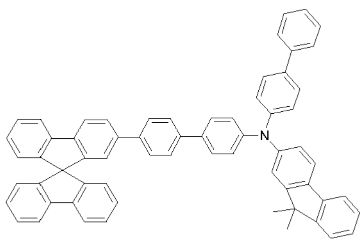 | 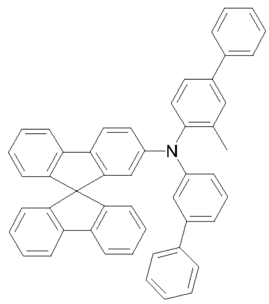 | 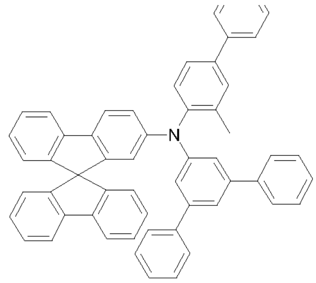 |
| (82) | (83) | (84) |
| 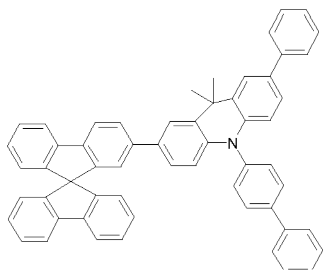 | 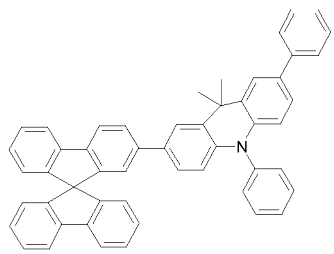 | 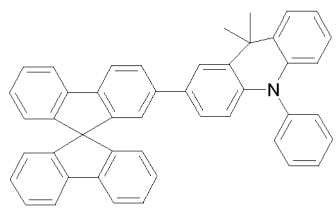 |
| (85) | (86) | (87) |
| 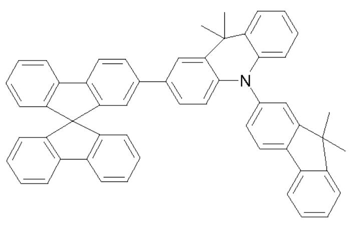 | 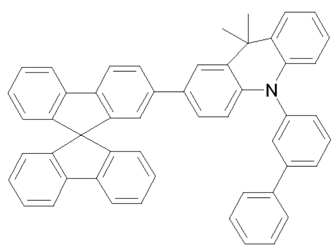 | 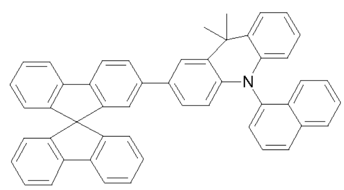 |
| (88) | (89) | (90) |
| 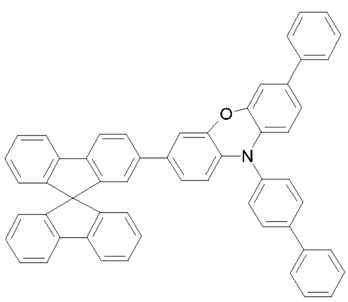 | 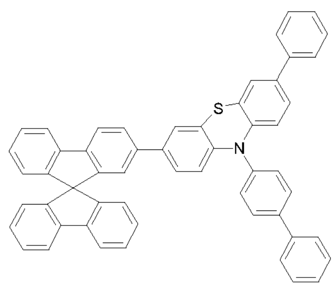 | 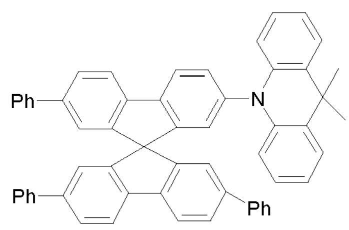 |
| (91) | (92) | (93) |

(fortgesetzt)

| | | |
|---|---|---|
| 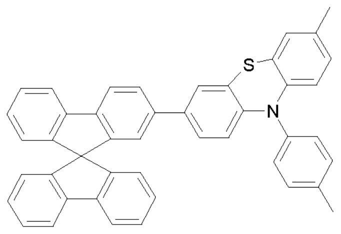 | 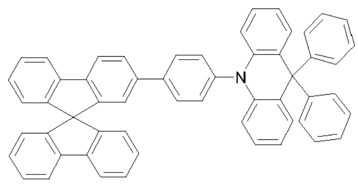 | 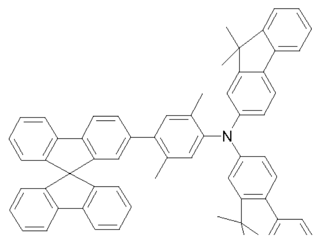 |
| (94) | (95) | (96) |
| 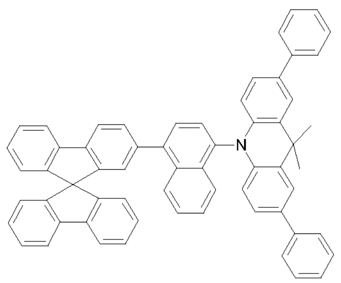 | 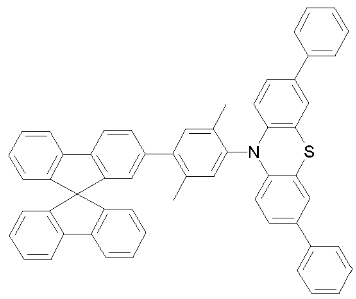 | 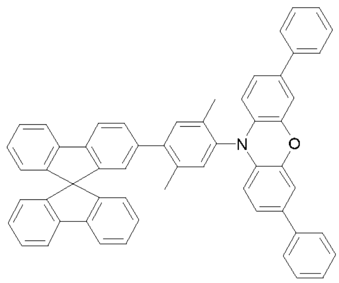 |
| (97) | (98) | (99) |
| 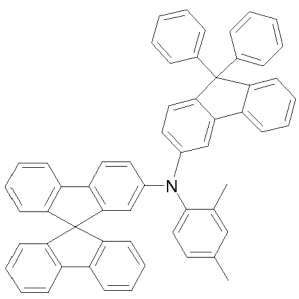 | 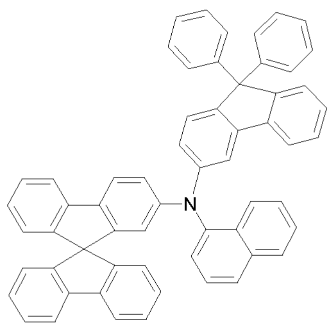 | 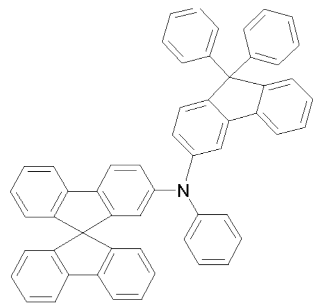 |
| (100) | (101) | (102) |
| 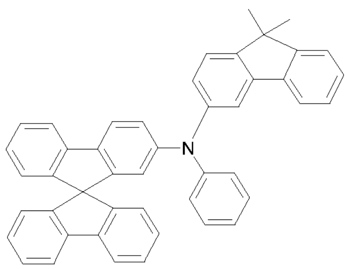 | 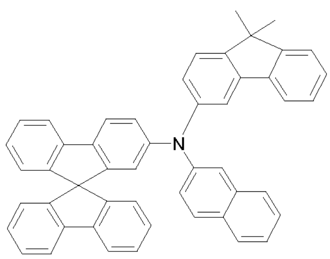 | 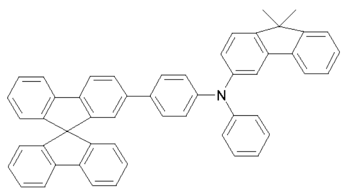 |
| (103) | (104) | (105) |
| 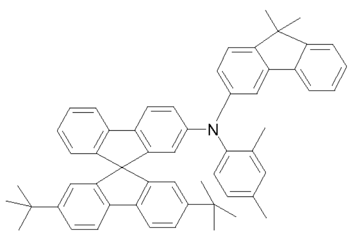 | 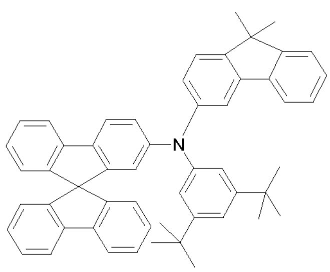 | 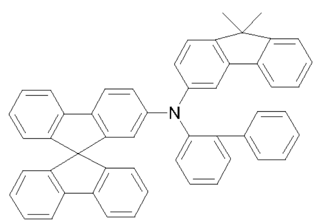 |
| (106) | (107) | (108) |

(fortgesetzt)

| | | |
|---|---|---|
| 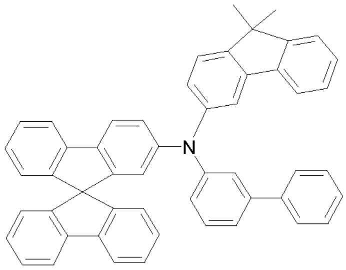 | 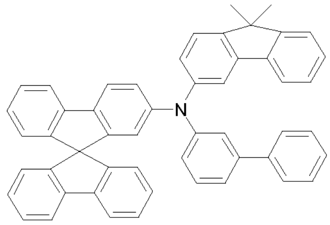 | 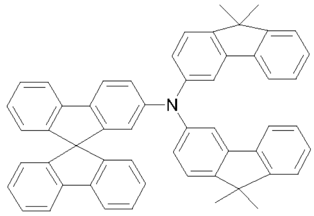 |
| (109) | (110) | (111) |
| 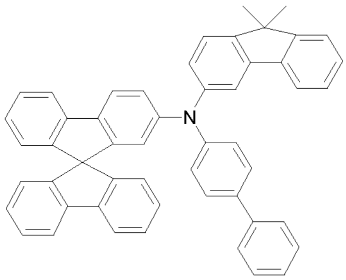 | 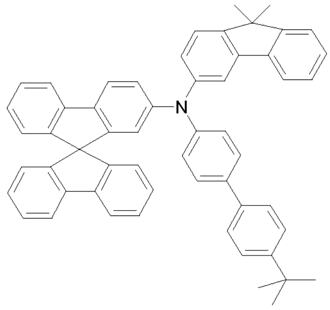 | 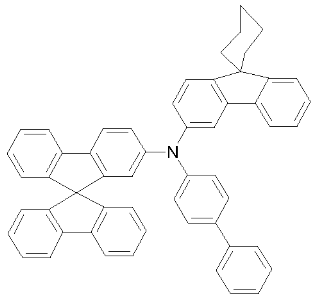 |
| (112) | (113) | (114) |
| 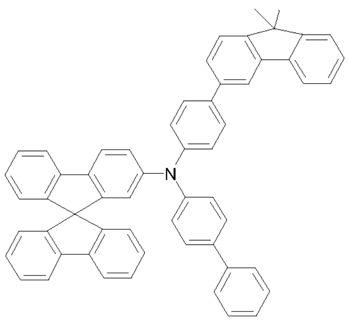 | 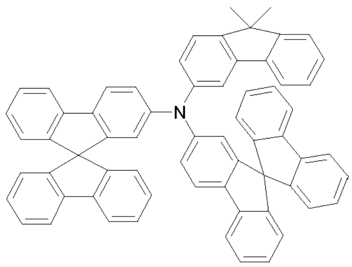 | 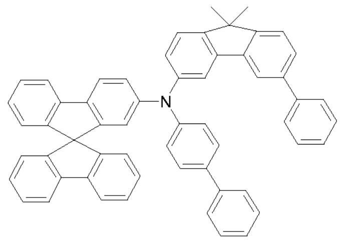 |
| (115) | (116) | (117) |
| 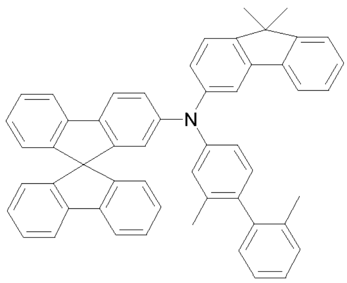 | 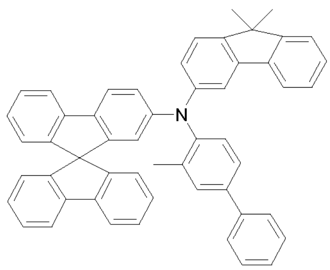 | 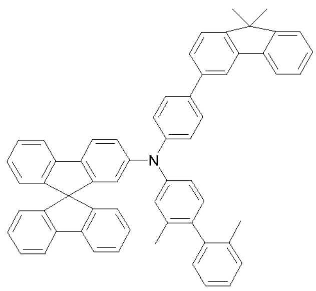 |
| (118) | (119) | (120) |
| 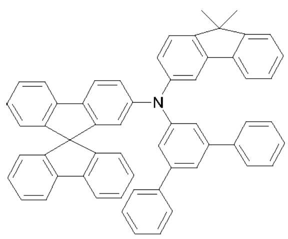 | 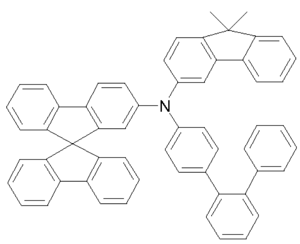 | 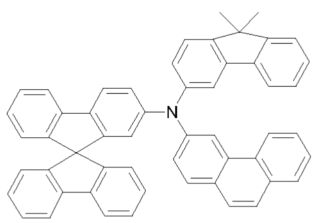 |
| (121) | (122) | (123) |

(fortgesetzt)

| | | |
|---|---|---|
| 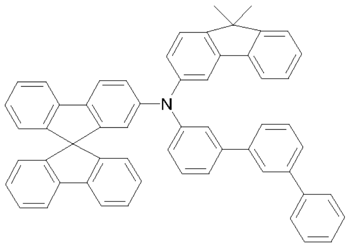 | 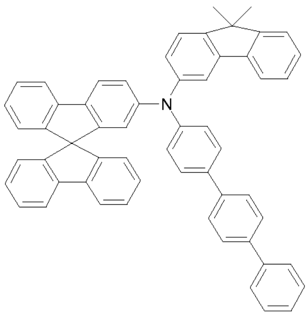 | 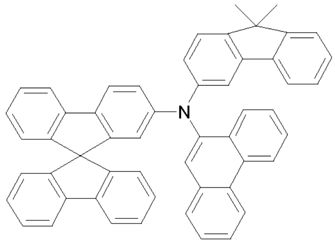 |
| (124) | (125) | (126) |
| 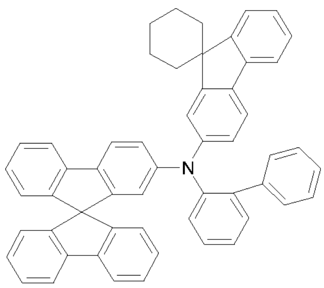 | 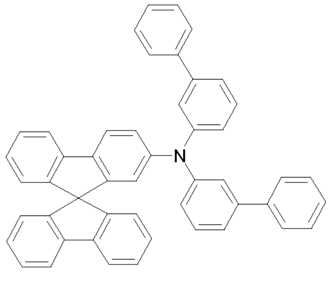 | 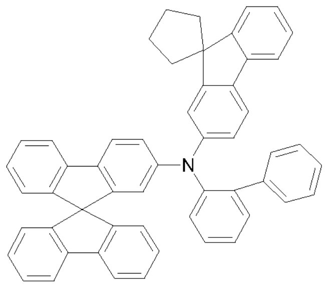 |
| (127) | (128) | (129) |
| 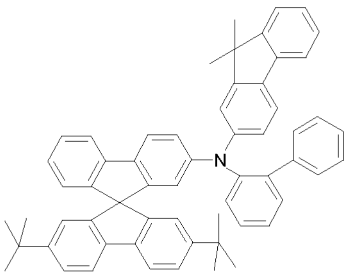 | 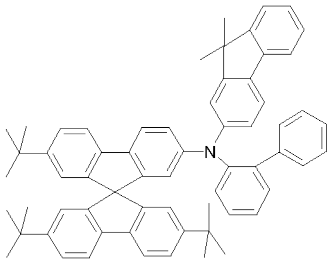 | 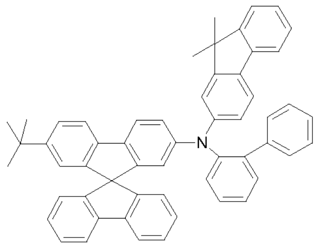 |
| (130) | (131) | (132) |
| 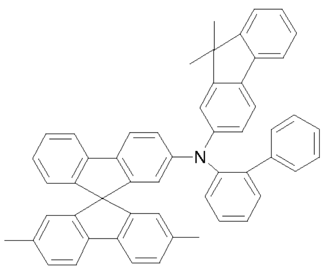 | 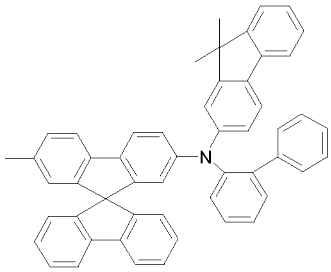 | 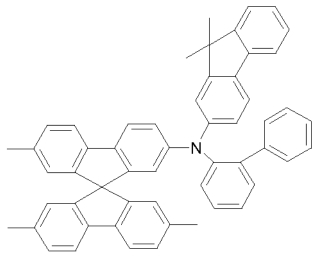 |
| (133) | (134) | (135) |
| 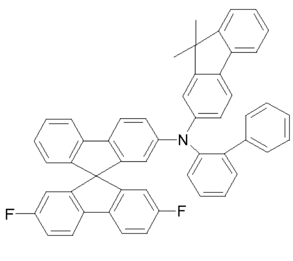 | 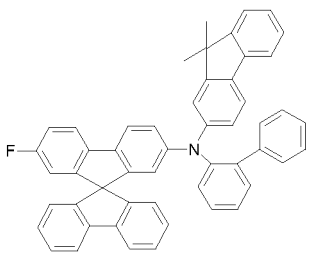 | 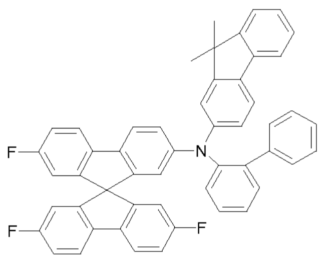 |
| (136) | (137) | (138) |

(fortgesetzt)

| | | |
|---|---|---|
| 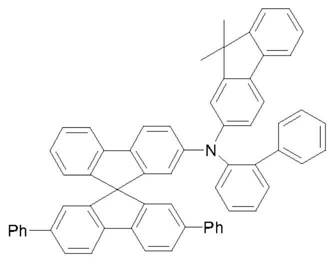 | 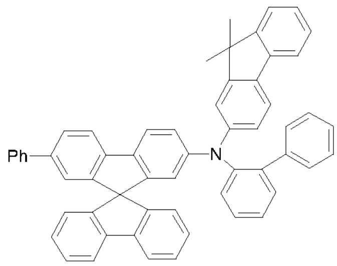 | 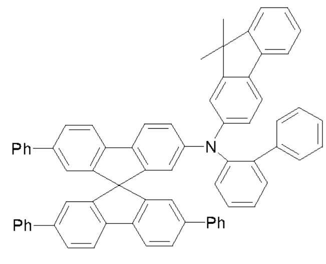 |
| (139) | (140) | (141) |
| 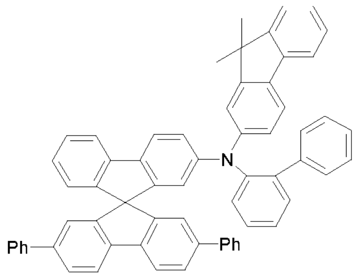 | 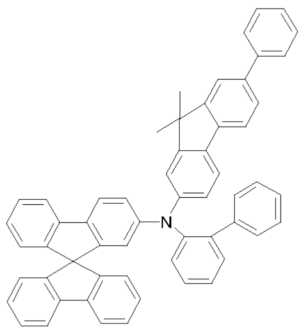 | 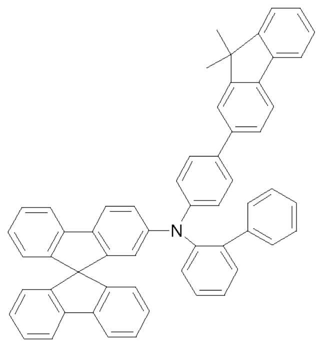 |
| (142) | (143) | (144) |
| 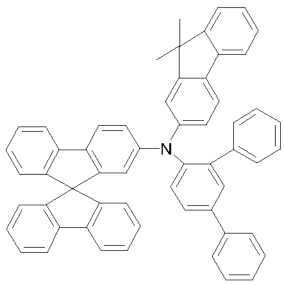 | 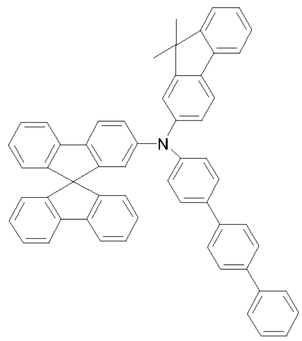 | 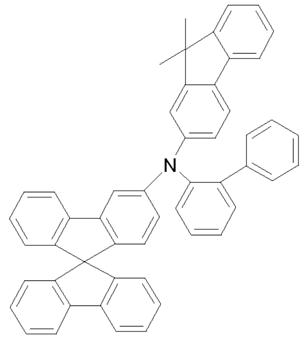 |
| (145) | (146) | (147) |
| 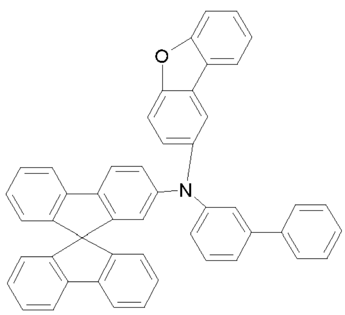 | 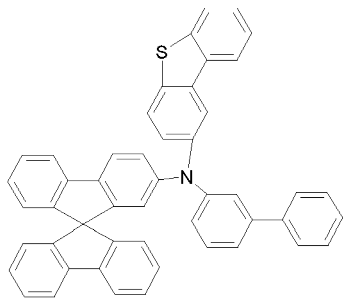 | 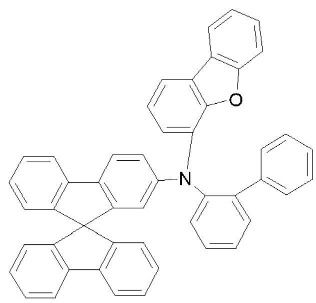 |
| (148) | (149) | (150) |

(fortgesetzt)

| | | |
|---|---|---|
| 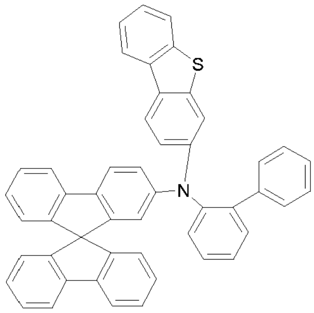 | 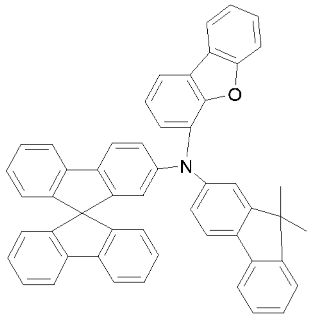 | 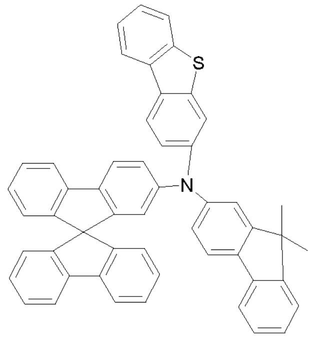 |
| (151) | (152) | (153) |
| 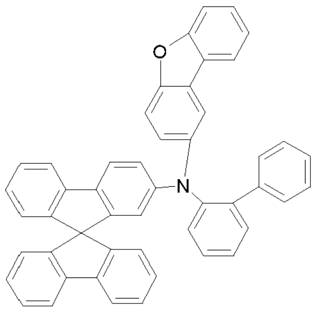 | 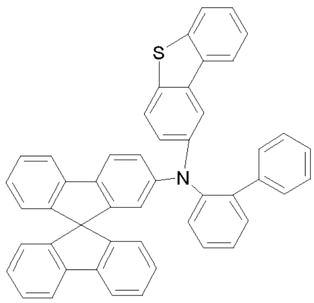 | 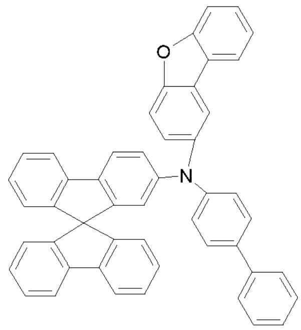 |
| (154) | (155) | (156) |
| 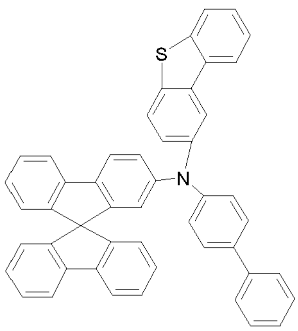 | 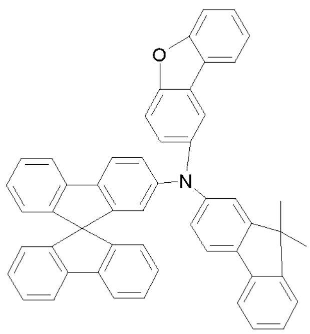 | 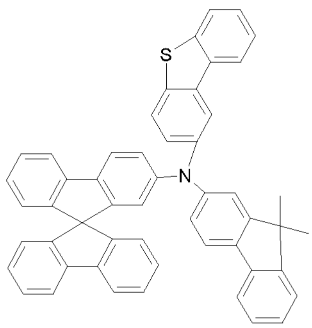 |
| (157) | (158) | (159) |
| 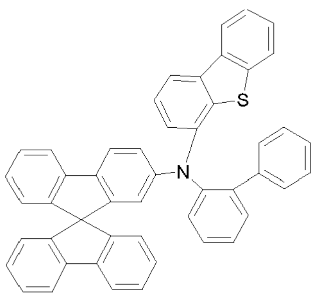 | 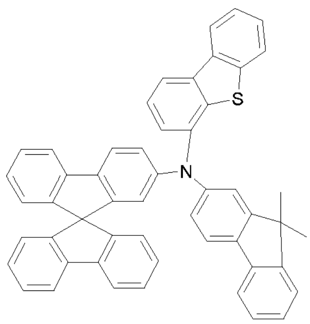 | 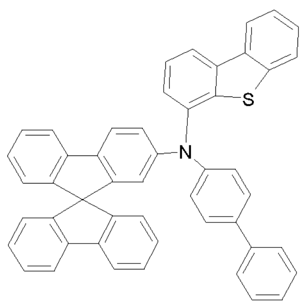 |
| (160) | (161) | (162) |

(fortgesetzt)

| | | |
|---|---|---|
| 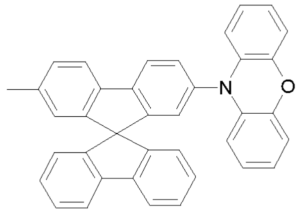 | 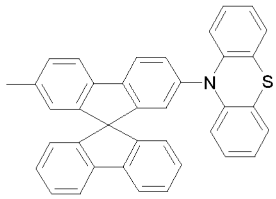 | 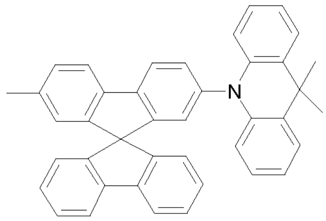 |
| (163) | (164) | (165) |
| 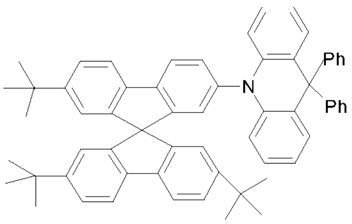 | 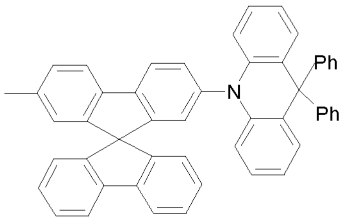 | 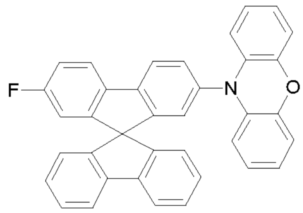 |
| (166) | (167) | (168) |
| 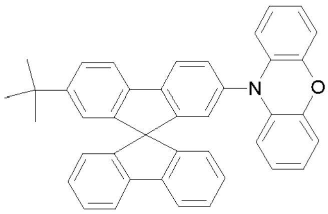 | 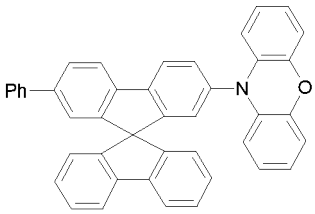 | 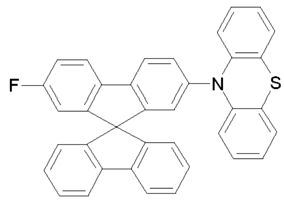 |
| (169) | (170) | (171) |
| 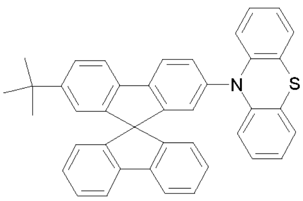 | 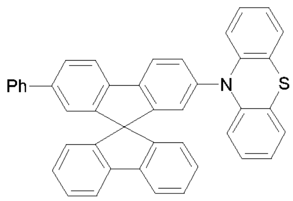 | 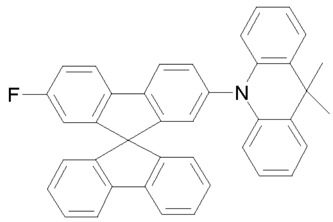 |
| (172) | (173) | (174) |
| 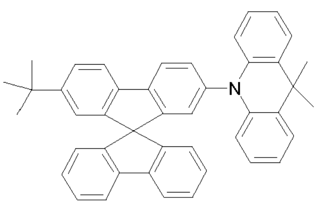 | 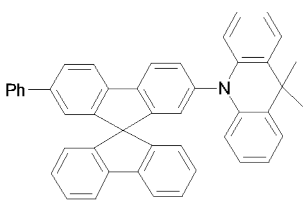 | 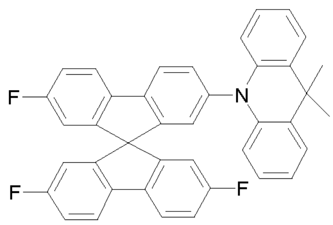 |
| (175) | (176) | (177) |
| 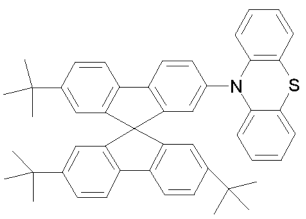 | 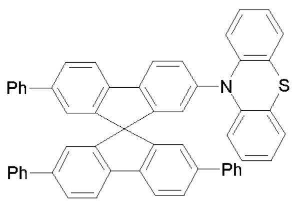 | 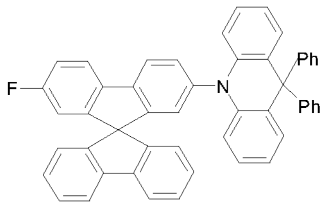 |
| (178) | (179) | (180) |

(fortgesetzt)

| | | |
|---|---|---|
| 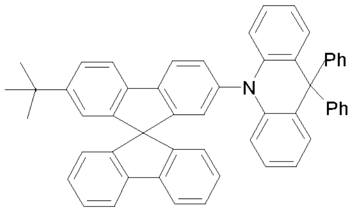 | 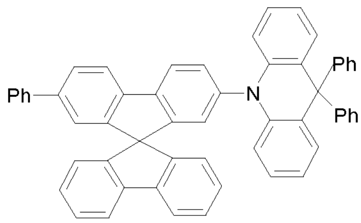 | 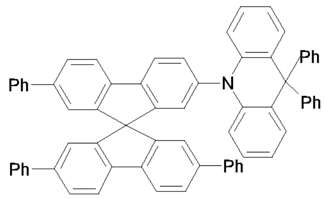 |
| (181) | (182) | (183) |
| 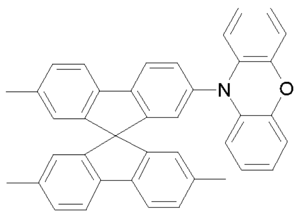 | 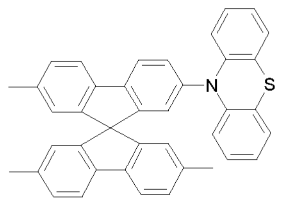 | 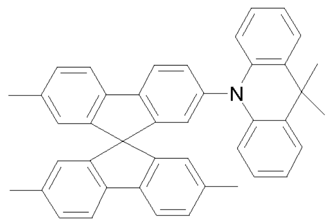 |
| (184) | (185) | (186) |
| 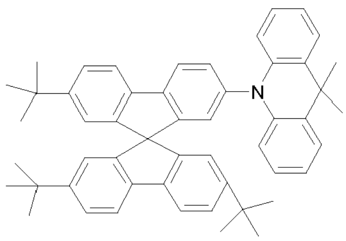 | 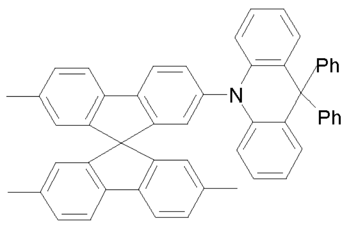 | 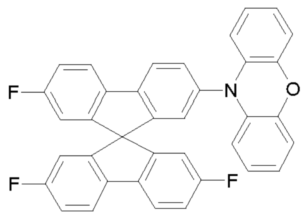 |
| (187) | (188) | (189) |
| 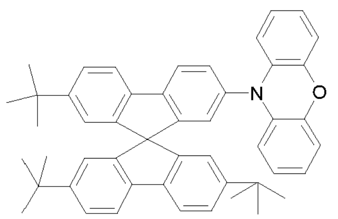 | 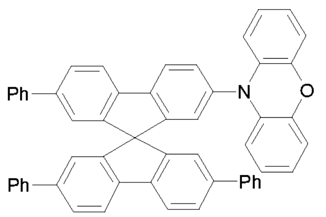 | 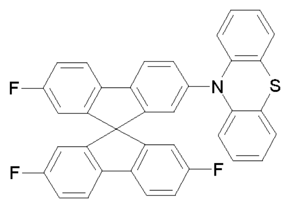 |
| (190) | (191) | (192) |
| 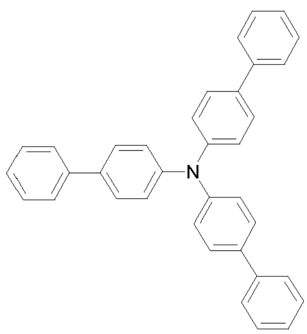 | 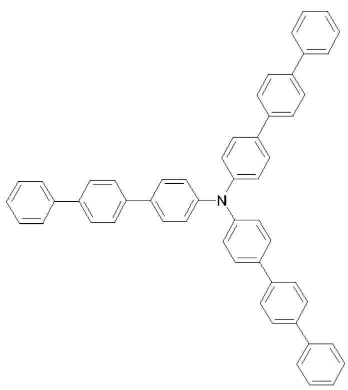 | 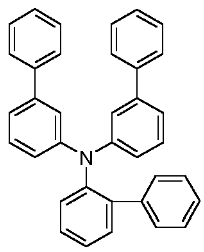 |
| (193) | (194) | (195) |

(fortgesetzt)

| | | |
|---|---|---|
| 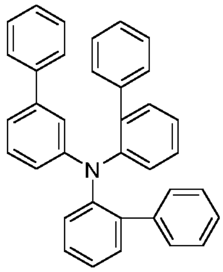 | 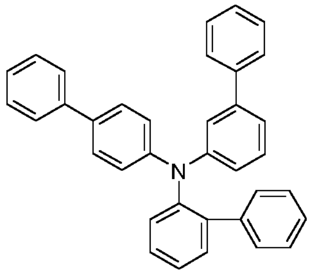 | 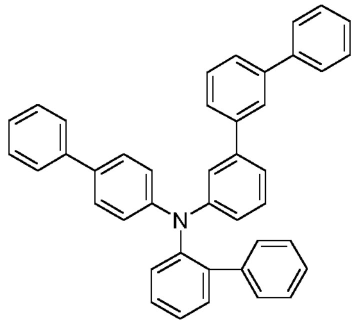 |
| (196) | (197) | (198) |
| 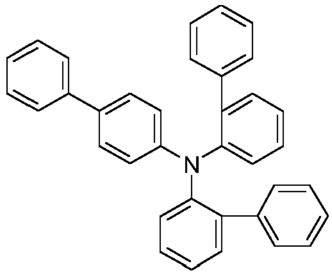 | 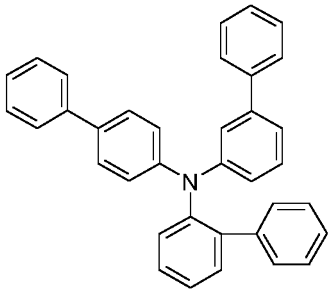 | 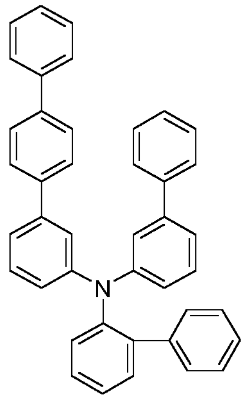 |
| (199) | (200) | (201) |
| 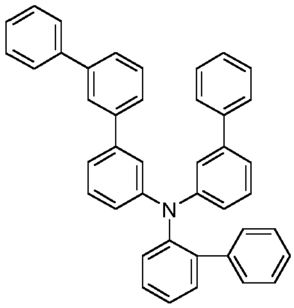 | 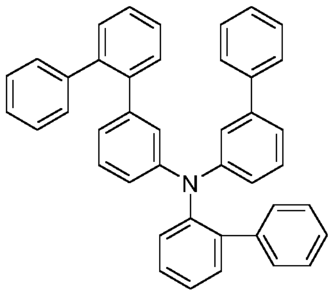 | 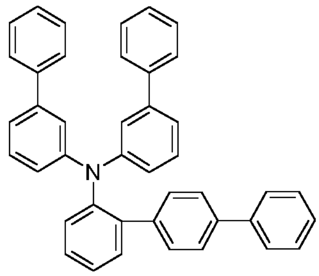 |
| (202) | (203) | (204) |
| 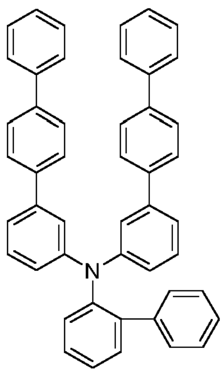 | 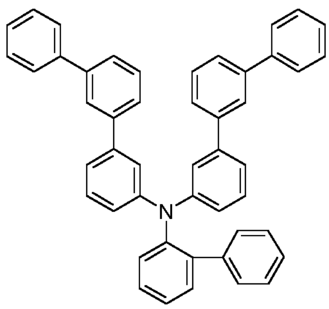 | 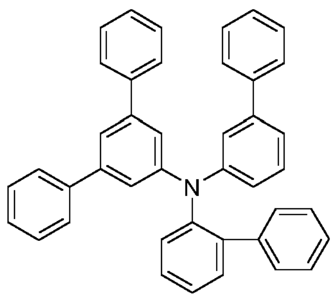 |
| (205) | (206) | (207) |

(fortgesetzt)

| | | |
|---|---|---|
| 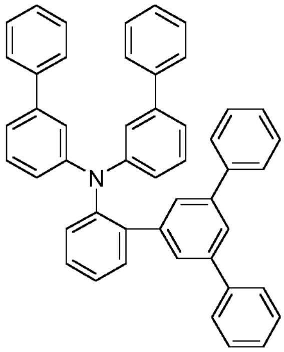 | 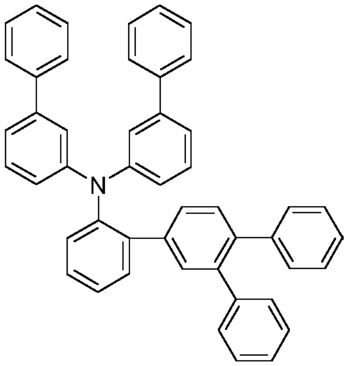 | 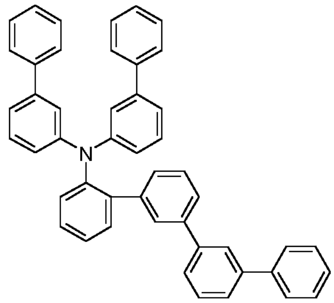 |
| (208) | (209) | (210) |
| 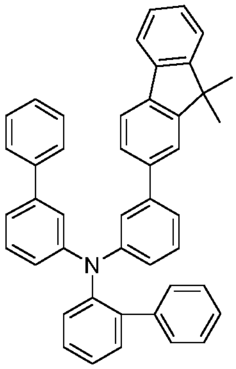 | 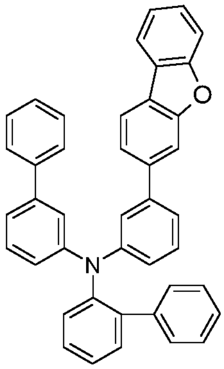 | 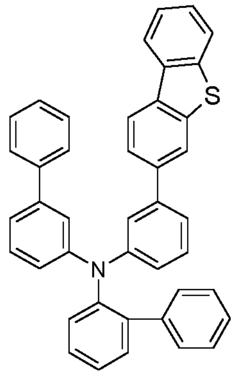 |
| (211) | (212) | (213) |
| 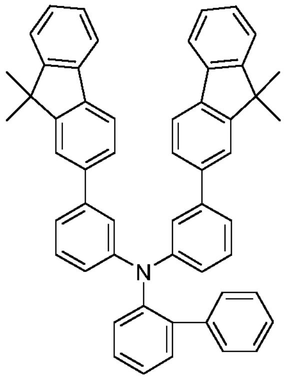 | 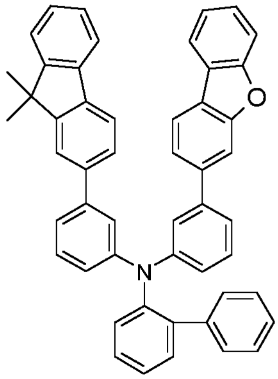 | 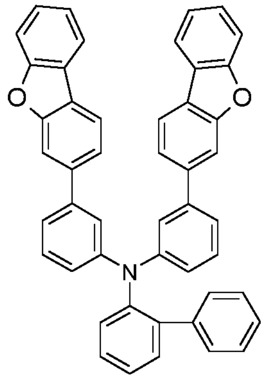 |
| (214) | (215) | (216) |
| 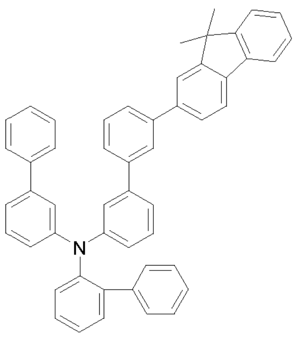 | 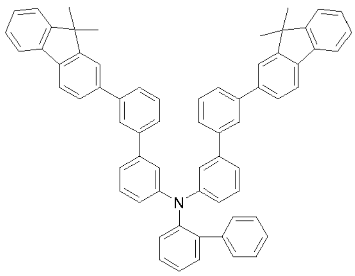 | 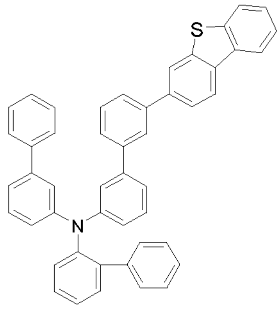 |
| (217) | (218) | (219) |

(fortgesetzt)

| | | |
|---|---|---|
| 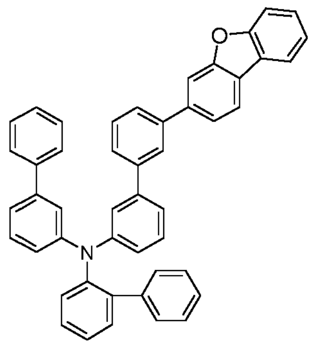 | 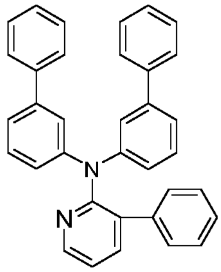 | 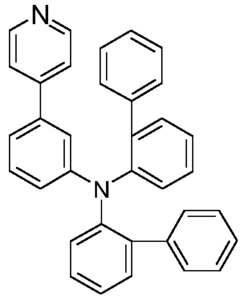 |
| (220) | (221) | (222) |
| 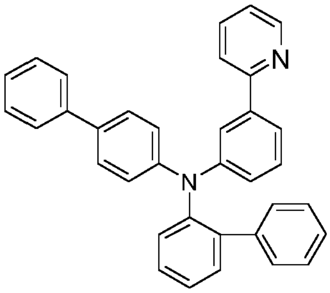 | 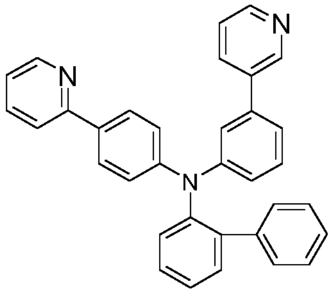 | 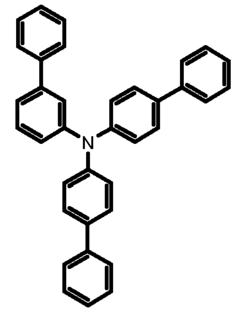 |
| (223) | (224) | (225) |
| 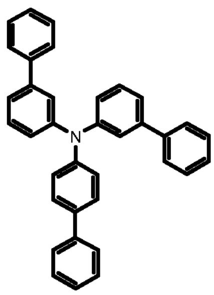 | 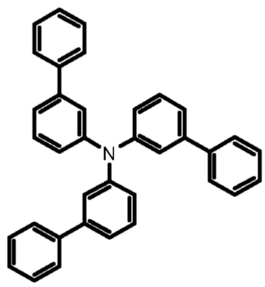 | 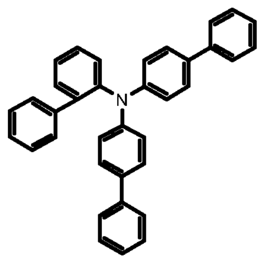 |
| (226) | (227) | (228) |
| 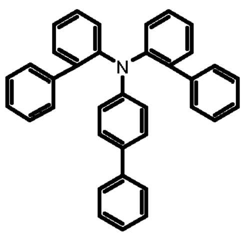 | 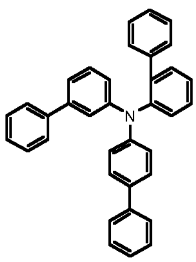 | 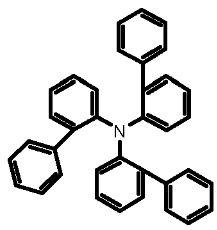 |
| (229) | (230) | (231) |

(fortgesetzt)

| | | |
|---|---|---|
| 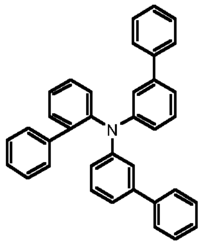 | 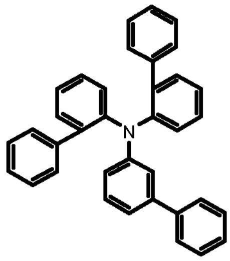 | 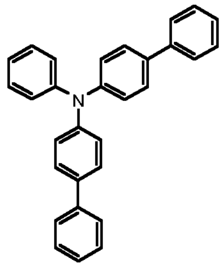 |
| (232) | (233) | (234) |
| 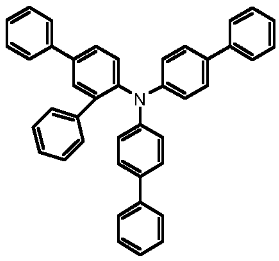 | 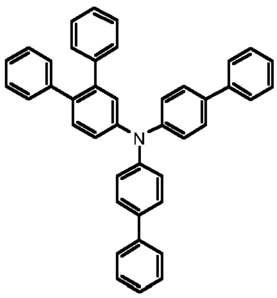 | 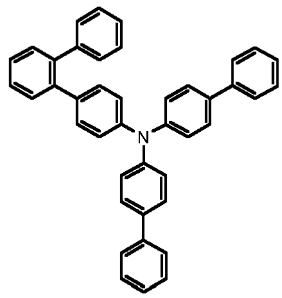 |
| (235) | (236) | (237) |
| 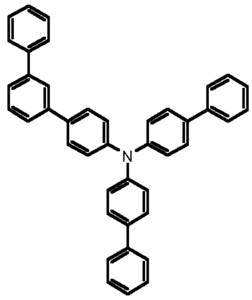 | 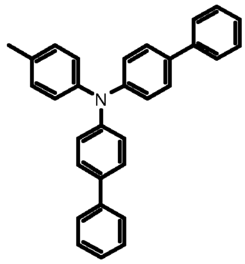 | 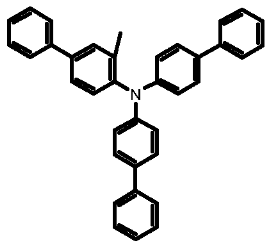 |
| (238) | (239) | (240) |
| 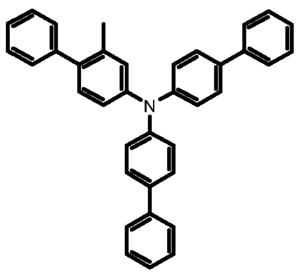 | 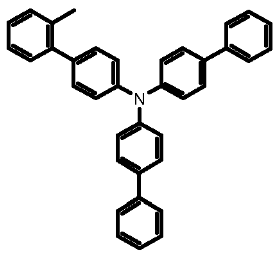 | 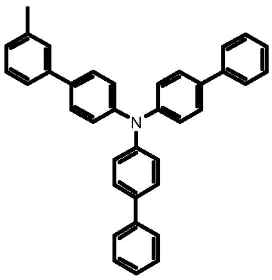 |
| (241) | (242) | (243) |
| 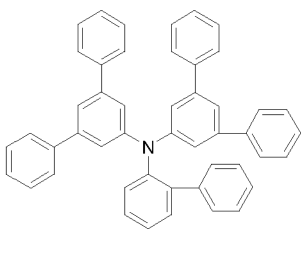 | 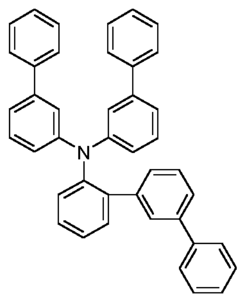 | 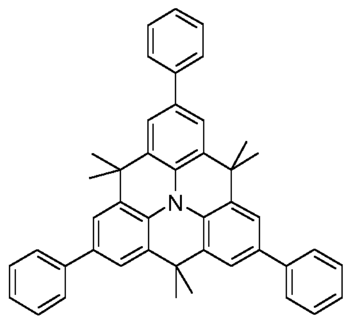 |

(fortgesetzt)

| (244) | (245) | (246) |
|---|---|---|
| 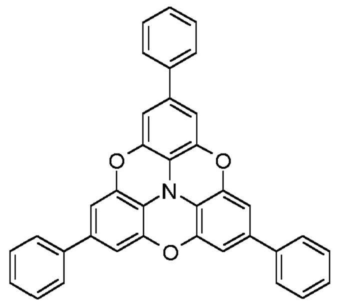 | 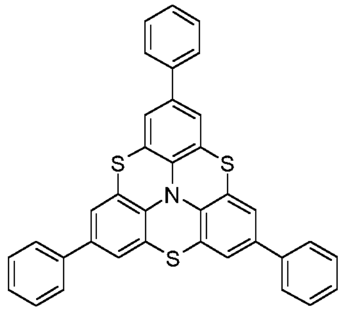 | 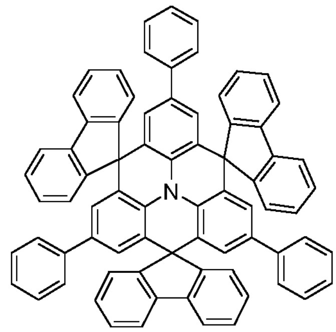 |
| (247) | (248) | (249) |
| 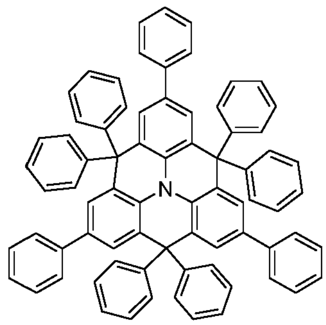 | 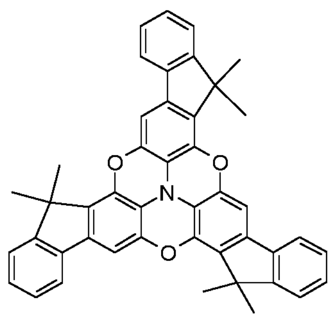 | 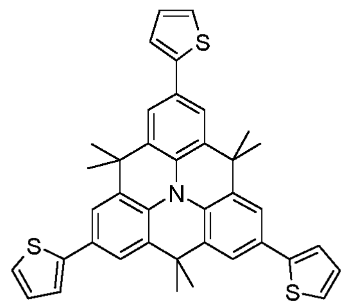 |
| (250) | (251) | (252) |
| 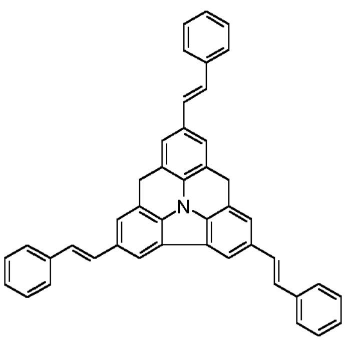 | 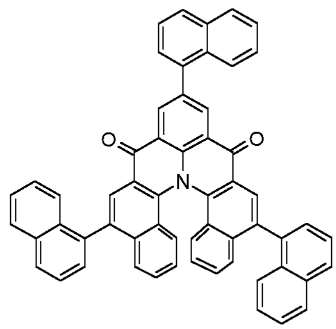 | 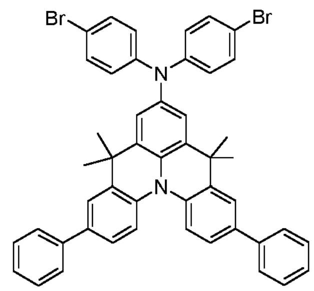 |
| (253) | (254) | (255) |

Die erfindungsgemäße elektronische Vorrichtung ist bevorzugt gewählt aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs). Besonders bevorzugt ist die elektronische Vorrichtung eine organische Elektrolumineszenzvorrichtung.

Die emittierende Schicht der elektronischen Vorrichtung kann eine fluoreszierende oder eine phosphoreszierende emittierende Schicht sein. Es liegt eine und es können mehrere emittierende Schichten in der Vorrichtung vorliegen.

Die erfindungsgemäße elektronische Vorrichtung kann mehrere emittierende Schichten enthalten. Besonders bevorzugt weisen diese emittierenden Schichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B. WO 2005/011013). Es soll angemerkt werden, dass sich für die Erzeugung von weißem

Licht anstelle mehrerer farbig emittierender Emitterverbindungen auch eine einzeln verwendete Emitterverbindung eignen kann, welche in einem breiten Wellenlängenbereich emittiert.

Die emittierende Schicht bzw. die emittierenden Schichten enthalten je eine oder mehrere Emitterverbindungen, die phosphoreszierend oder fluoreszierend sein können.

Vom Begriff phosphoreszierende Emitter sind typischerweise Verbindungen umfasst, bei denen die Lichtemission durch einen spinverbotenen Übergang erfolgt, beispielsweise einen Übergang aus einem angeregten Triplettzustand oder einem Zustand mit einer höheren Spinquantenzahl, beispielsweise einem Quintett-Zustand.

Als phosphoreszierende Emitter eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten. Bevorzugt werden als phosphoreszierende Dotanden Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium, Platin oder Kupfer enthalten.

Dabei werden im Sinne der vorliegenden Erfindung alle lumineszierenden Iridium-, Platin- oder Kupferkomplexe als phosphoreszierende Verbindungen angesehen.

Beispiele für phosphoreszierende Emitter können den Anmeldungen WO 2000/70655, WO 2001/41512, WO 2002/02714, WO 2002/15645, EP 1191613, EP 1191612, EP 1191614, WO 2005/033244, WO 2005/019373 und US 2005/0258742 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind, zur Verwendung in den erfindungsgemäßen Vorrichtungen. Auch kann der Fachmann ohne erfinderisches Zutun weitere phosphoreszierende Komplexe in Kombination mit den erfindungsgemäßen Verbindungen in OLEDs einsetzen.

Konkrete Beispiele für phosphoreszierende Emitter, die in der erfindungsgemäßen elektronischen Vorrichtung eingesetzt werden können, sind im Folgenden aufgeführt.

| | | |
|---|---|---|
| 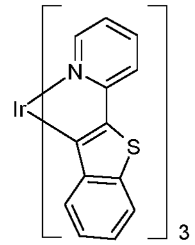 | 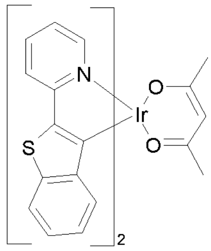 | 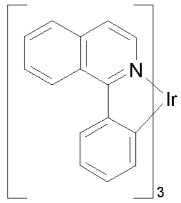 |
| 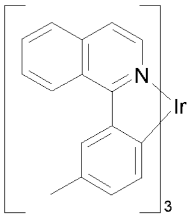 | 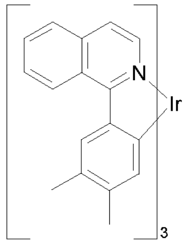 | 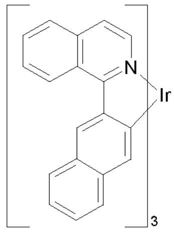 |
| 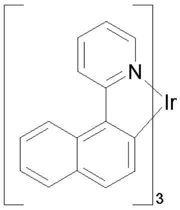 | 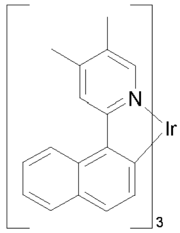 | 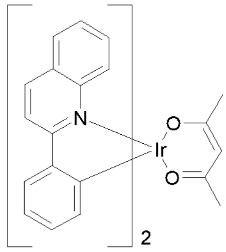 |
| 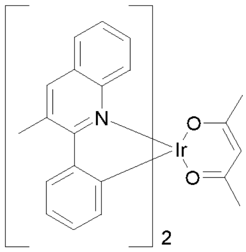 | 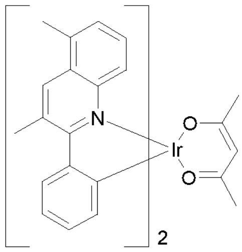 | 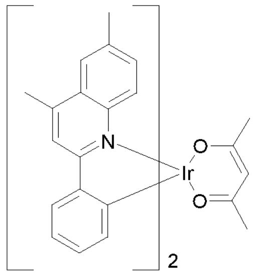 |
| 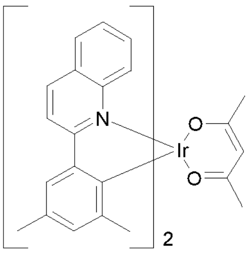 | 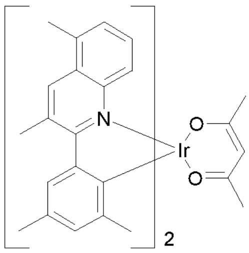 | 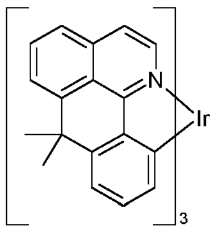 |
| 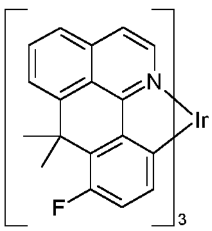 | 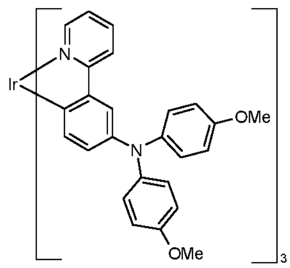 | 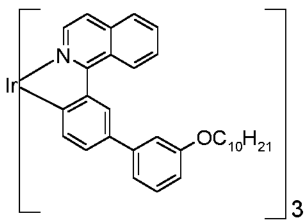 |

| | | |
|---|---|---|
| 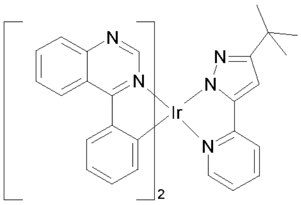 | 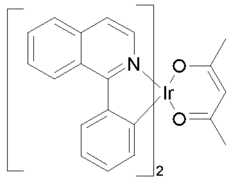 | 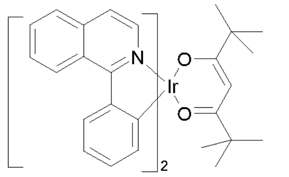 |
| 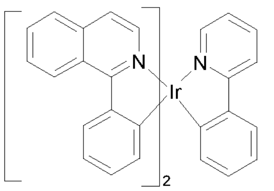 | 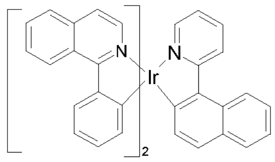 | 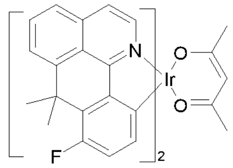 |
| 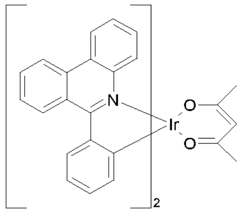 | 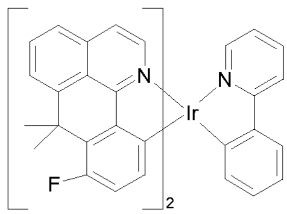 | 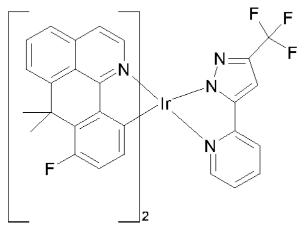 |
| 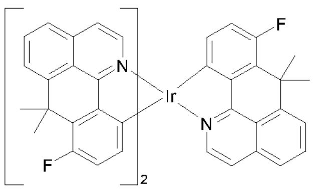 | 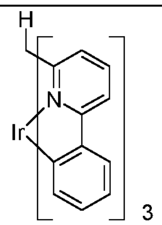 | 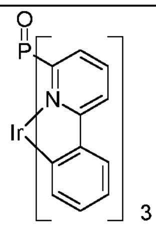 |
| 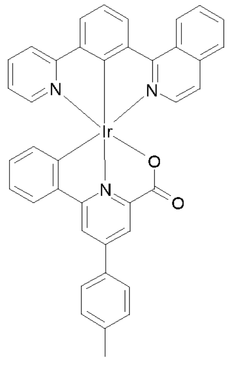 | 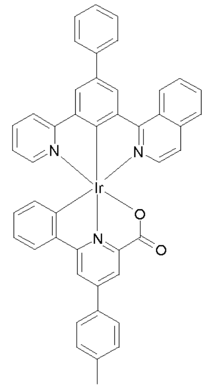 | 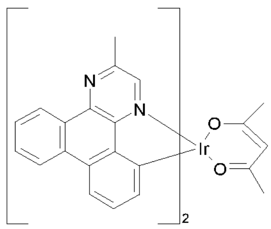 |
| 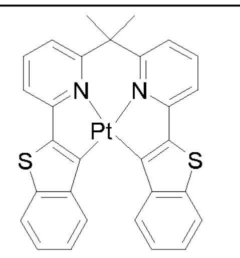 | 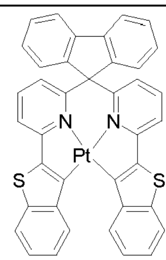 | 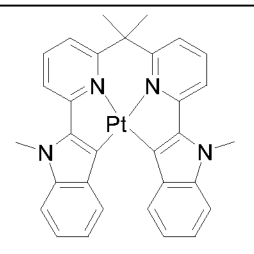 |

| | | |
|---|---|---|
| 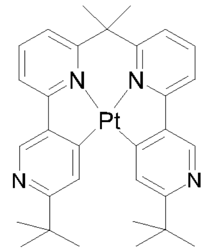 | 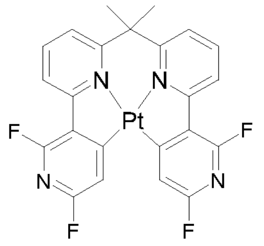 | 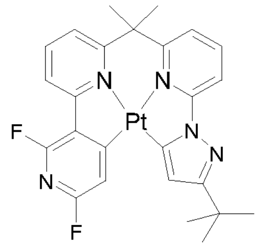 |
| 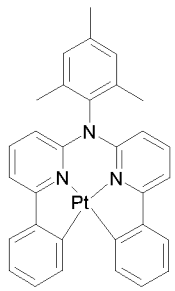 | 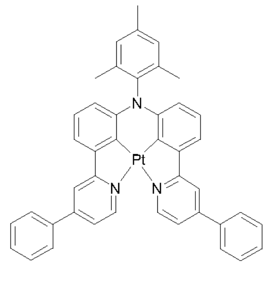 | 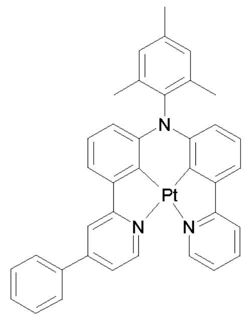 |
| 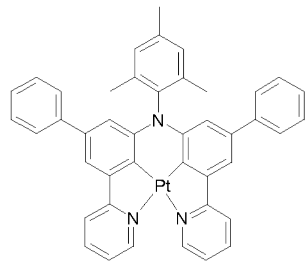 | 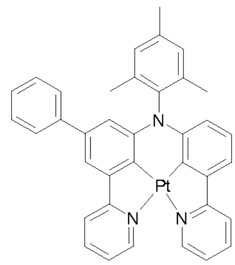 | 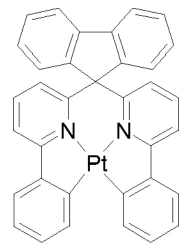 |
| 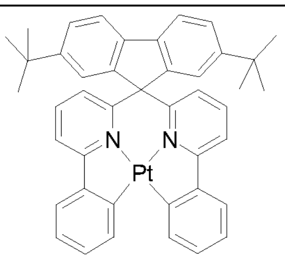 | 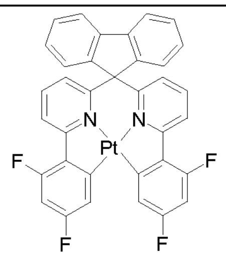 | 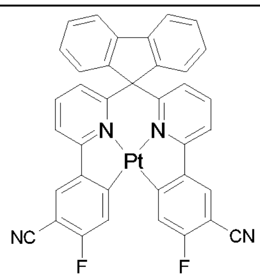 |
| 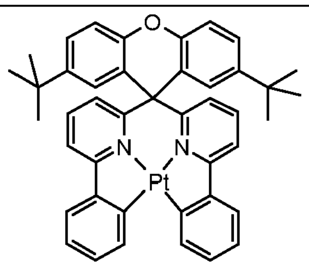 | 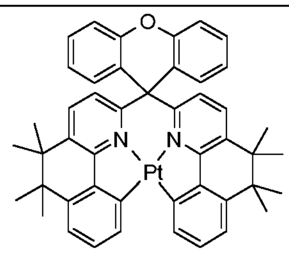 | 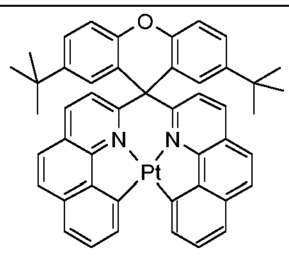 |

| | | |
|---|---|---|
| 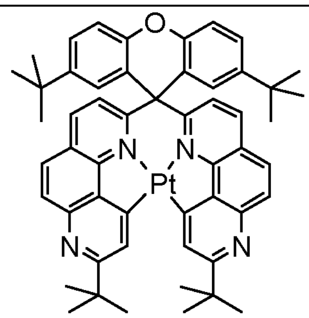 | 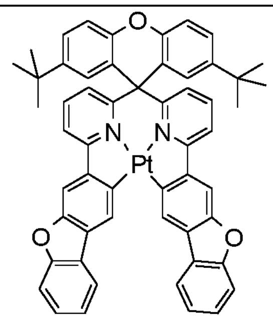 | 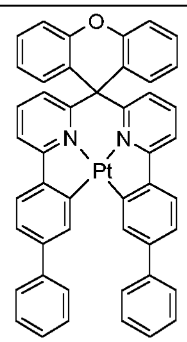 |
| 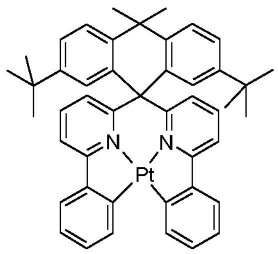 | 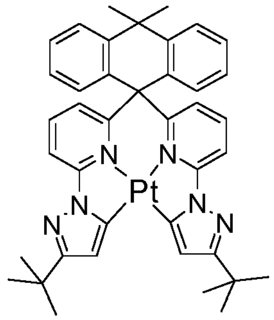 | 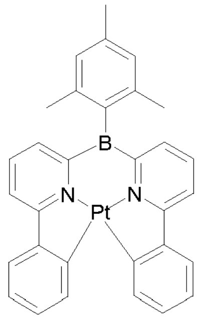 |
| 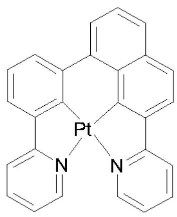 | 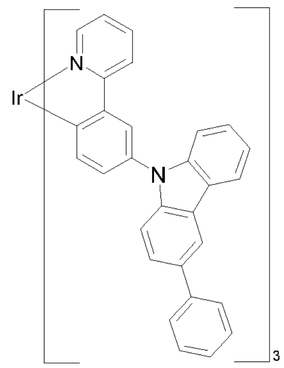 | 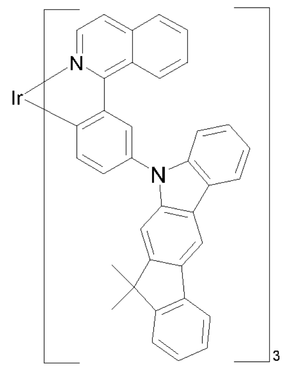 |
| 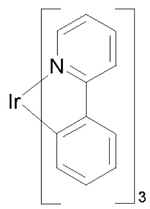 | 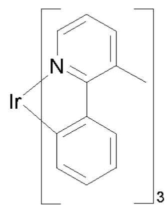 | 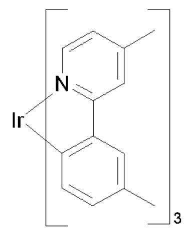 |
| 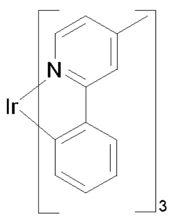 | 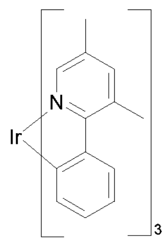 | 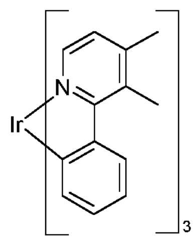 |

| | | |
|---|---|---|
| 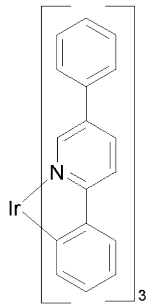 | 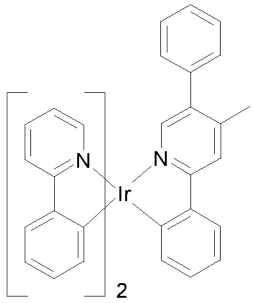 | 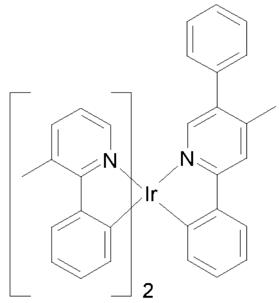 |
| 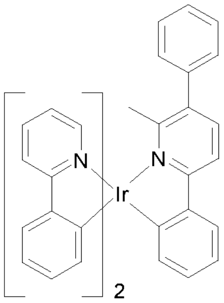 | 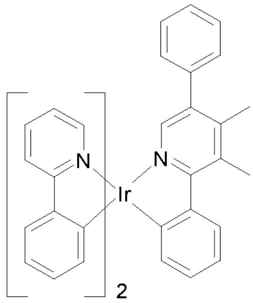 | 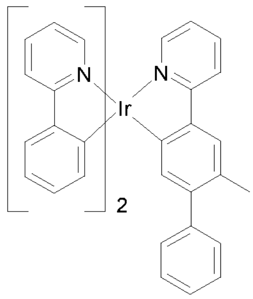 |
| 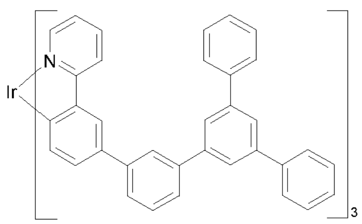 | 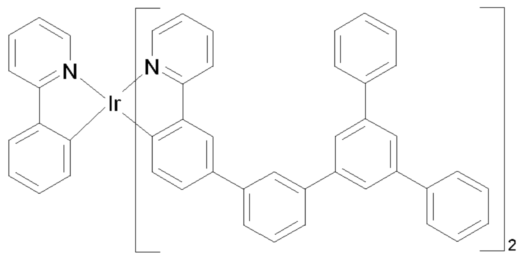 | |
| 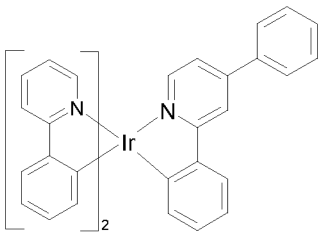 | 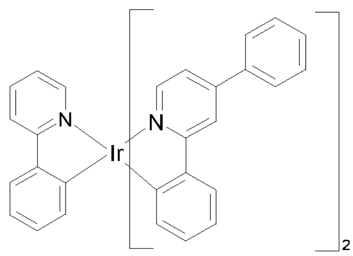 | 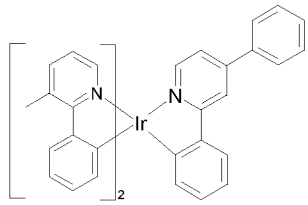 |
| 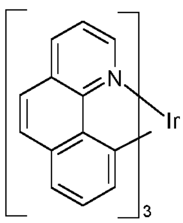 | 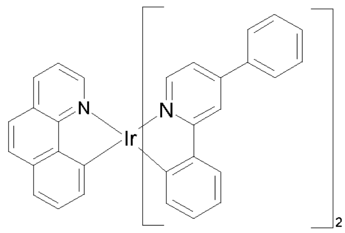 | 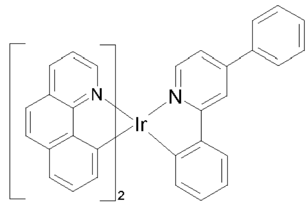 |

| | | |
|---|---|---|
| 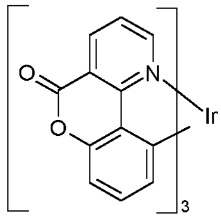 | 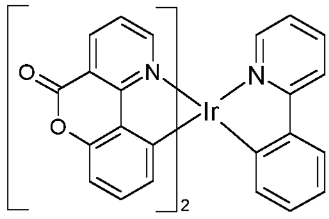 | 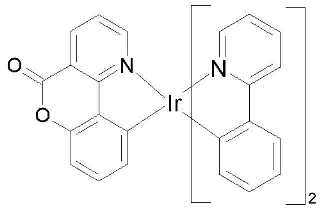 |
| 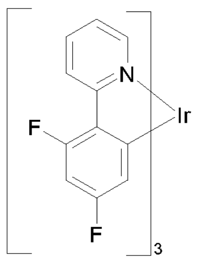 | 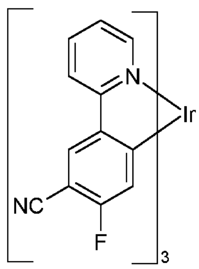 | 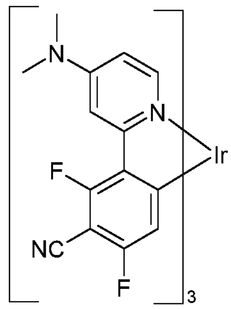 |
| 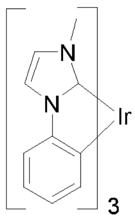 | 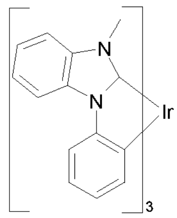 | 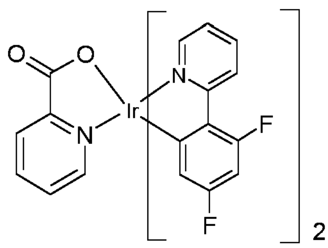 |
| 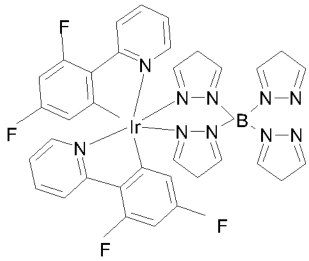 | 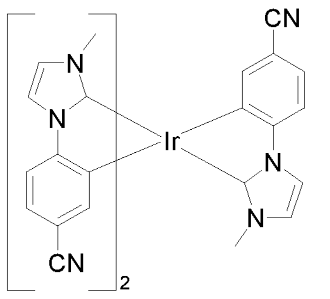 | 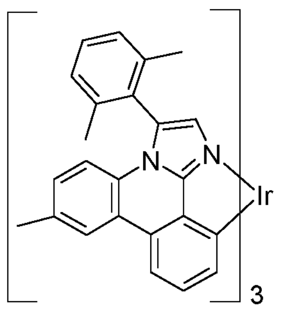 |
| 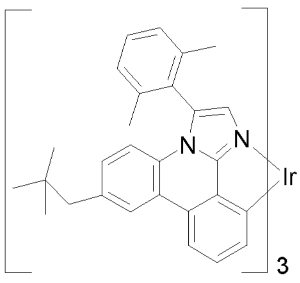 | 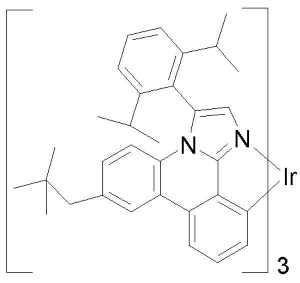 | 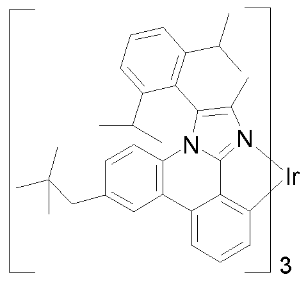 |

| | | |
|---|---|---|
| 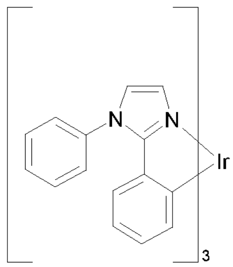 | 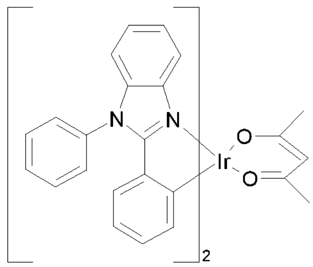 | 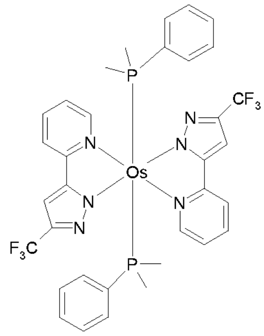 |
| 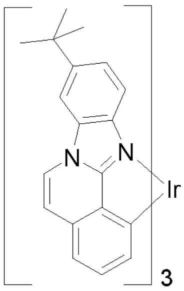 | 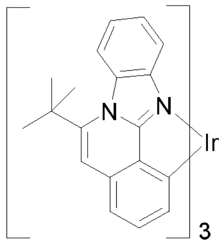 | 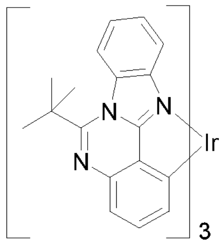 |
| 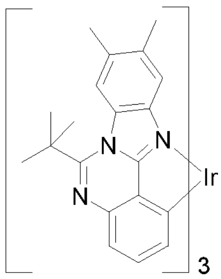 | 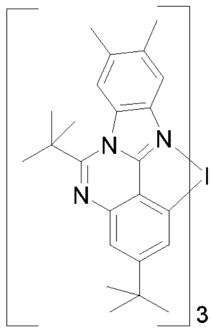 | 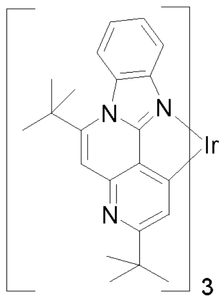 |

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Die emittierende Schicht enthält neben einer oder mehreren emittierenden Verbindungen ein Mono-Triarylamin als Host und bevorzugt ein weiteres oder weitere Matrixmaterialien.

Als Matrixmaterialien, bevorzugt für fluoreszierende Emitter, kommen Materialien verschiedener Stoffklassen in Frage. Bevorzugte Matrixmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 2004/081017), der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239). Besonders bevorzugte Matrixmaterialien sind ausgewählt aus den Klassen der Oligoarylene, enthaltend Naphthalin, Anthracen, Benzanthracen und/oder Pyren oder

Atropisomere dieser Verbindungen, der Oligoarylenvinylene, der Ketone, der Phosphinoxide und der Sulfoxide. Ganz besonders bevorzugte Matrixmaterialien sind ausgewählt aus den Klassen der Oligoarylene, enthaltend Anthracen, Benzanthracen, Benzphenanthren und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Matrixmaterialien für phosphoreszierende Emitter sind aromatische Amine, insbesondere Triarylamine, z. B. gemäß US 2005/0069729, Carbazolderivate (z. B. CBP, N,N-Biscarbazolylbiphenyl) oder Verbindungen gemäß WO 2005/039246, US 2005/0069729, JP 2004/288381, EP 1205527 oder WO 2008/086851, verbrückte Carbazolderivate, z. B. gemäß WO 2011/088877 und WO 2011/128017, Indenocarbazolderivate, z. B. gemäß WO 2010/136109 und WO 2011/000455, Azacarbazolderivate, z. B. gemäß EP 1617710, EP 1617711, EP 1731584, JP 2005/347160, Indolocarbazolderivate, z. B. gemäß WO 2007/063754 oder WO 2008/056746, Ketone, z. B. gemäß WO 2004/093207 oder WO 2010/006680, Phosphinoxide, Sulfoxide und Sulfone, z. B. gemäß WO 2005/003253, Oligophenylene, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß WO 2010/015306, WO 2007/063754 oder WO 2008/056746, Zinkkomplexe, z. B. gemäß EP 652273 oder WO 2009/062578, Aluminiumkomplexe, z. B. BAlq, Diazasilol- und Tetraazasilol-Derivate, z. B. gemäß WO 2010/054729, DiazaphospholDerivate, z. B. gemäß WO 2010/054730 und Aluminiumkomplexe, z. B. BAIQ.

Geeignete Ladungstransportmaterialien, wie sie in der Lochinjektions- bzw. Lochtransportschicht bzw. Elektronenblockierschicht oder in der Elektronentransportschicht der erfindungsgemäßen elektronischen Vorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderivate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956, WO 2004/028217, WO 2004/080975 und WO 2010/072300 offenbart werden.

Allgemein bevorzugt sind als Lochtransportmaterialien, die in einer Lochtransport-, Lochinjektions- oder Elektronenblockierschicht in der erfindungsgemäßen Elektrolumineszenzvorrichtung verwendet werden können, Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß den noch nicht offengelegten Anmeldungen EP 12005369.9, EP 12005370.7 und EP 12005371.5), Spiro-Dibenzopyran-Amine (z. B. gemäß der noch nicht offengelegten Anmeldung EP 11009127.9) und Dihydroacridin-Derivate (z. B. gemäß der noch nicht offen gelegten EP 11007067.9).

Als Kathode der elektronischen Vorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.).

Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. $Al/Ni/NiO_x$, $Al/PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

Die Herstellung der elektronischen Vorrichtung erfolgt bevorzugt dadurch, dass eine oder mehrere Schichten durch Sublimation erzeugt werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Insbesondere wird die p-dotierte Schicht A bevorzugt durch Co-Sublimation von Dotand und Mono-Triarylamin hergestellt. Ein Verfahren hierzu ist beispielsweise in Solar Energy Materials & Solar Cells, 2000, 63, 83-99, beschrieben.

Bevorzugt ist ebenfalls, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist es, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen erforderlich. Ausreichende Löslichkeit lässt sich durch geeignete Substitution der Verbindungen erreichen.

Weiterhin bevorzugt ist es, dass zur Herstellung der erfindungsgemäßen elektronischen Vorrichtung eine oder mehrere Schichten aus Lösung und eine oder mehrere Schichten durch ein Sublimationsverfahren hergestellt werden.

Die Vorrichtung wird bei der Herstellung (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

Folgende Abbildungen erläutern die vorliegende Erfindung:

Fig. 1* zeigt eine elektronische Vorrichtung (1), enthaltend Anode (2), Schicht A (3), Schicht B (4), emittierende Schicht (5), Elektronentransportschicht (6) und Kathode (7).

Fig. 2* zeigt eine elektronische Vorrichtung (1), enthaltend Anode (2), Schicht A (3), Schicht B (4), emittierende Schicht (5), Elektronentransportschicht (6), Elektroneninjektionsschicht (6a) und Kathode (7).

Fig. 3 zeigt eine elektronische Vorrichtung (1), enthaltend Anode (2), Schicht A (3), Schicht B (4), Schicht C (4a), emittierende Schicht (5), Elektronentransportschicht (6), Elektroneninjektionsschicht (6a) und Kathode (7).

Fig. 4* zeigt eine elektronische Vorrichtung (1), enthaltend Anode (2), Schicht A' (2a), Schicht A (3), Schicht B (4), emittierende Schicht (5), Elektronentransportschicht (6), Elektroneninjektionsschicht (6a) und Kathode (7).

Die vorstehenden und im folgenden mit * markierten Abbildungen und Ausführungsbeispiele sind nicht anspruchsgemäß.

## Ausführungsbeispiele

### A) Bestimmung der HOMO/LUMO-Lagen von Verbindungen

Die HOMO- und LUMO-Lagen der Materialien werden über quantenchemische Rechnungen bestimmt. Hierzu wird das Programmpaket "Gaussian03W" (Gaussian Inc.) verwendet. Zur Berechnung organischer Substanzen ohne Metalle wird zuerst eine Geometrieoptimierung mit der Methode "Ground State/Semiempirical/Default Spin/AM1/Charge 0/Spin Singlet" durchgeführt. Im Anschluss erfolgt auf Grundlage der optimierten Geometrie eine Energierechnung. Hierbei wird die Methode "TD-SFC/DFT/Default Spin/B3PW91" mit dem Basissatz "6-31G(d)" verwendet (Charge 0, Spin Singlet). Für metallorganische Verbindungen wird die Geometrie über die Methode "Ground State/Hartree-Fock/Default Spin/LanL2MB/Charge 0/Spin Singlet" optimiert. Die Energierechnung erfolgt analog zu den organischen Substanzen wie oben beschrieben mit dem Unterschied, dass für das Metallatom der Basissatz "LanL2DZ" und für die Liganden der Basissatz "6-31G(d)" verwendet wird. Aus der Energierechnung erhält man das HOMO HEh bzw. LUMO LEh in Hartree-Einheiten. Daraus werden die anhand von Cyclovoltammetriemessungen kalibrierten HOMO- und LUMO-Werte in Elektronenvolt wie folgt bestimmt:

$$HOMO(eV) = ((HEh*27.212)-0.9899)/1.1206$$

$$LUMO(eV) = ((LEh*27.212)-2.0041)/1.385$$

Diese Werte sind im Sinne dieser Anmeldung als HOMO bzw. LUMO der Materialien anzusehen. Die Werte für die in den Beispielen eingesetzten Materialien sind in Tabelle 1 zusammengefasst.

Tabelle 1: HOMO/LUMO-Werte der Materialien

| Material | HOMO (eV) | LUMO (eV) |
|---|---|---|
| CbzA1 | -5.18 | --- |
| SpA1 | -4.85 | --- |
| F4TCNQ | --- | -5.21 |
| MA1 | -5.26 | --- |
| MA2 | -5.25 | --- |
| TIFA1 | -5.01 | --- |
| NPB | -5.19 | --- |
| MA3 | -5.40 | --- |
| MA4 | -5.41 | --- |
| MA5 | -5.35 | --- |
| MA6 | -5.27 | --- |
| MA7 | -5.31 | --- |
| MA8 | -5.46 | --- |
| MA9 | -5.35 | --- |
| MA10 | -5.42 | --- |
| MA11 | -5.18 | --- |
| MA12 | -5.32 | --- |
| MA13 | -5.32 | --- |
| MA14 | -5.27 | --- |
| MA15 | -5.19 | --- |
| MA16 | -5.23 | --- |
| MA17 | -5.23 | --- |
| MA18 | -5.20 | --- |
| MA19 | -5.43 | --- |
| MA20 | -5.20 | --- |
| MA21 | -5.20 | --- |
| MA24 | -5.38 | --- |
| MA25 | -5.14 | --- |
| MA26 | -5.14 | --- |
| MA27 | -5.12 | --- |
| MA28 | -5.32 | --- |
| MA29 | -5.24 | --- |

**B) Herstellung der Verbindungen**

Die Herstellung der verwendeten Verbindungen kann gemäß dem allgemeinen Fachwissen erfolgen. Die Herstellung von MA1 ist beispielsweise in JP 1995-053955 offenbart. Verfahren zur Herstellung von MA2, MA6, MA7, MA11, MA20, MA25 und MA27 sind beispielsweise in WO 2012/034627 offenbart.

**C) Herstellung der OLEDs**

Die Herstellung von erfindungsgemäßen OLEDs sowie OLEDs nach dem Stand der Technik erfolgt nach einem allgemeinen Verfahren gemäß WO 2004/058911, das auf die hier beschriebenen Gegebenheiten (Schichtdickenvariation, Materialien) angepasst wird.

In den folgenden Beispielen V1 bis E46 (siehe Tabellen 2 und 3) werden die Daten verschiedener OLEDs vorgestellt. Glasplättchen, die mit strukturiertem ITO (Indium Zinn Oxid) der Dicke 50 nm beschichtet sind, bilden die Substrate für die OLEDs. Die Substrate werden nass gereinigt (Spülmaschine, Reiniger Merck Extran), anschließend 15 min lang bei 250°C ausgeheizt und vor der Beschichtung mit einem Sauerstoffplasma behandelt.

Auf die vorbehandelten Substrate werden verschiedene Schichten aufgebracht: Erste Lochtransportschicht (HTL1) / optionale zweite Lochtransportschicht (HTL2) / direkt an EML grenzende Lochtransportschicht (HTL3) / Emissionsschicht (EML) / Elektronentransportschicht (ETL) / optionale Elektroneninjektionsschicht (EIL) und abschließend eine 100 nm dicke Aluminiumkathode. Der genaue Aufbau der OLEDs ist Tabelle 2 zu entnehmen. Die zur Herstellung der OLEDs benötigten Materialien sind in Tabelle 4 gezeigt.

In den Beispielen E16*, E44 ist zwischen EML und ETL zusätzlich eine 10nm dicke Schicht des Materials IC1 enthalten.

In den Beispielen E42, E43 ist zwischen EML und ETL zusätzlich eine 10nm dicke Schicht des Materials IC2 enthalten.

In den Beispielen E15* und E16* ist zwischen ITO und HTL1 zusätzlich eine 20nm dicke Schicht PEDOT:PSS enthalten (Poly(3,4-ethylenedioxythiophene) poly(styrenesulfonate), bezogen als CLEVIOS™ P VP AI 4083 von Heraeus Precious Metals GmbH Deutschland, aus wässriger Lösung aufgeschleudert).

Die weiteren Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie M1:D1 (95%:5%) bedeutet hierbei, dass das Material M1 in einem Volumenanteil von 95% und D1 in einem Volumenanteil von 5% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik sowie die Lebensdauer bestimmt. Die Elektrolumineszenzspektren werden bei einer Leuchtdichte von 1000 $cd/m^2$ bestimmt und daraus die CIE 1931 x und y Farbkoordinaten berechnet. Die Angabe U1000 in Tabelle 3 bezeichnet die Spannung, die für eine Leuchtdichte von 1000 $cd/m^2$ benötigt wird. SE1000 und LE1000 bezeichnen die Strom- bzw. Leistungseffizienz, die bei 1000 $cd/m^2$ erreicht werden. EQE1000 schließlich bezeichnet die externe Quanteneffizienz bei einer Betriebsleuchtdichte von 1000 $cd/m^2$. Als Lebensdauer LD wird die Zeit definiert, nach der die Leuchtdichte bei Betrieb mit konstanter Stromdichte (in Tabelle 3 in Spalte "$j_{LD}$" angegeben) von der Startleuchtdichte auf einen gewissen Anteil A abfällt.

Die Daten der verschiedenen OLEDs sind in Tabelle 3 zusammengefasst. Die Beispiele V1-V16 sind Vergleichsbeispiele gemäß dem Stand der Technik, die Beispiele E1-E3, E5-E17 und E46 zeigen Daten von OLEDs, und die Beispiele E4 und E18-E45 zeigen Daten von erfindungsgemäßen OLEDs.

Im folgenden werden einige der Beispiele näher erläutert, um die Vorteile der erfindungsgemäßen OLEDs zu verdeutlichen. Es sei jedoch darauf hingewiesen, dass dies nur eine Auswahl der in Tabelle 3 gezeigten Daten darstellt.

Verglichen mit einer OLED mit p-dotiertem Monoamin als HTL1 und dem Diamin NPB als HTL3 angrenzend an die EML zeigt eine OLED mit gleicher HTL1 und dem Monoamin MA2 als HTL3 wesentlich bessere Effizienz und Lebensdauer sowie leicht bessere Spannung (Beispiele V1 und E9).

Die Beispiele mit dem blau fluoreszierenden Dotanden D2 (V2-V7, V10, E5*-E11*, E14*) zeigen, dass die Kombination eines p-dotierten Monoamins als HTL1 und eines Monoamins angrenzend an die EML die besten Ergebnisse liefert. Werden Diamine, Amin-Carbazole oder Tetraamine nach dem Stand der Technik als HTL1, HTL2 oder direkt an die EML angrenzend als HTL3 verwendet, sind die Leistungsdaten weniger gut. Bei Verwendung einer p-dotierten Schicht des Monoamins MA2 in Kombination mit dem p-dotierten Tetraamin SpA1 sowie MA2 direkt angrenzend an die EML erhält man ebenfalls sehr gute Leistungsdaten (Beispiel E14*).

Verwendet man in Kombination mit dem blauen Dotanden D1 das Carbazol-Amin CbzA1 in der HTL3 (also in

direktem Kontakt zur EML) und das p-dotierte Monoamin MA1, so erhält man höhere Spannung und schlechtere Effizienz und Lebensdauer als bei Verwendung der Monoamine MA1 bzw. MA2 in der HTL3 (Beispiele E1*, E2* und E12). Das gleiche gilt, wenn man statt des blau fluoreszierenden Dotanden D1 den phosphoreszenten grünen Dotanden TEG1 verwendet (Beispiele E3*, E4 und E13).

Man erhält besonders gute Lebensdauern, wenn das Monoamin, welches p-dotiert wird, auch in den anderen Lochtransportschichten eingesetzt wird (Vergleich Beispiele E1* und E2* sowie E3* und E4).

Bei sehr guter Elektroneninjektion ist weiterhin die Verwendung des rein Phenyl substituierten Monoamins MA1 gegenüber dem Monoamin MA2 vorteilhaft (Vergleich Beispiele E10* und E11*). Die gezeigten OLEDs zeigen zwar geringere Lebensdauern, dafür aber niedrigere Spannungen als vergleichbare OLEDs mit geringerer Elektroneninjektion, was für einige Anwendungen vorteilhaft sein kann.

Die Beispiele E18 bis E45 und E46* zeigen, dass als Mono-Triarylamin gemäß der vorliegenden Erfindung eine Vielzahl an unterschiedlichen Verbindungen ohne Änderung des grundlegenden technischen Vorteils der Erfindung eingesetzt werden können. Die Verbindungen MA1 bis MA29 umfassen unter anderem Fluorenyl-Mono-Triarylamine, Xanthen-Mono-Triarylamine, Spirobifluoren-Mono-Triarylamine, Indenofluoren-Mono-Triarylamine und weitere Substanzklassen. In den Tabellen 2 und 3 handelt es sich bei den Beispielen E4 und E18 bis E45 um erfindungsgemäße Beispiele. Die mit einem Stern gekennzeichneten Beispiele E1 bis E3, E5 bis E11, E14 bis E17 und E46, sowie die nicht mit einem Stern gekennzeichneten Beispiele E12 und E13 fallen nicht unter die Ansprüche.

Tabelle 2: Aufbau der OLEDs

| Bsp | HTL1 Dicke | HTL2 Dicke | HTL3 Dicke | EML Dicke | ETL Dicke | EIL Dicke |
|---|---|---|---|---|---|---|
| V1 | MA2:F4TCNQ (97%:3%) 140nm | --- | NPB 20nm | M1:D2 (95%:5%) 20nm | ST1:LiQ (50%:50%) 30nm | --- |
| V2 | SpA1:F4TCNQ (96%:4%) 10nm | --- | NPB 170nm | M1:D2 (95%:5%) 20nm | ST1:LiQ (50%:50%) 30nm | LiQ 1nm |
| V3 | CbzA1:F4TCNQ (96%:4%) 10nm | --- | CbzA1 170nm | M1:D2 (95%:5%) 20nm | ST1:LiQ (50%:50%) 30nm | LiQ 1nm |
| V4 | CbzA1:F4TCNQ (96%:4%) 10nm | --- | CbzA1 170nm | M1:D2 (95%:5%) 20nm | ST1 30nm | LiQ 3nm |
| V5 | SpA1:F4TCNQ (96%:4%) 10nm | SpA1 140nm | MA2 30nm | M1:D2 (95%:5%) 20nm | ST1:LiQ (50%:50%) 30nm | LiQ 1nm |
| V6 | CbzA1:F4TCNQ (96%:4%) 170nm | --- | CbzA1 10nm | M1:D2 (95%:5%) 20nm | ST1:LiQ (50%:50%) 30nm | LiQ 1nm |
| V7 | CbzA1:F4TCNQ (96%:4%) 170nm | --- | CbzA1 10nm | M1:D2 (95%:5%) 20nm | ST1 30nm | LiQ 3nm |
| V8 | SpA1:F4TCNQ (96%:4%) 160nm | --- | CbzA1 20nm | M1:D1 (95%:5%) 20nm | ST1:LiQ (50%:50%) 30nm | LiQ 1nm |
| V9 | SpA1:F4TCNQ (96%:4%) 170nm | --- | CbzA1 70nm | IC1:TEG1 (90%:10%) 30nm | ST1:LiQ (50%:50%) 40nm | LiQ 1nm |
| V10 | SpA1:F4TCNQ (96%:4%) 10nm | SpA1 140nm | MA2 30nm | M1:D2 (95%:5%) 20nm | ST1:LiQ (50%:50%) 30nm | LiQ 1nm |
| V11 | CbzA1:F4TCNQ (96%:4%) 20nm | CbzA1 175nm | MA2 20nm | M1:D1 (95%:5%) 20nm | ST1:LiQ (50%:50%) 30nm | LiQ 1nm |
| V12 | CbzA1:F4TCNQ (96%:4%) 20nm | CbzA1 175nm | MA6 20nm | M1:D1 (95%:5%) 20nm | ST1:LiQ (50%:50%) 30nm | LiQ 1nm |
| V13 | CbzA1:F4TCNQ (96%:4%) 20nm | CbzA1 175nm | MA7 20nm | M1:D1 (95%:5%) 20nm | ST1:LiQ (50%:50%) 30nm | LiQ 1nm |
| V14 | CbzA1:F4TCNQ (96%:4%) 20nm | CbzA1 175nm | MA8 20nm | M1:D1 (95%:5%) 20nm | ST1:LiQ (50%:50%) 30nm | LiQ 1nm |

(fortgesetzt)

| Bsp | HTL1 Dicke | HTL2 Dicke | HTL3 Dicke | EML Dicke | ETL Dicke | EIL Dicke |
|---|---|---|---|---|---|---|
| V15 | CbzA1:F4TCNQ (96%:4%) 20nm | CbzA1 175nm | MA4 20nm | M1:D1 (95%:5%) 20nm | ST1:LiQ (50%:50%) 30nm | LiQ 1nm |
| V16 | CbzA1:F4TCNQ (96%:4%) 20nm | CbzA1 175nm | MA5 20nm | M1:D1 (95%:5%) 20nm | ST1:LiQ (50%:50%) 30nm | LiQ 1nm |
| E1* | MA1:F4TCNQ (96%:4%) 20nm | --- | MA1 160nm | M1:D1 (95%:5%) 20nm | ST1:LiQ (50%:50%) 30nm | LiQ 1nm |
| E2* | MA1:F4TCNQ (96%:4%) 20nm | | MA2 160nm | M1:D1 (95%:5%) 20nm | ST1:LiQ (50%:50%) 30nm | LiQ 1nm |
| E3* | MA1:F4TCNQ (96%:4%) 20nm | --- | MA1 220nm | IC1:TEG1 (90%:10%) 30nm | ST1:LiQ (50%:50%) 30nm | LiQ 1nm |
| E4 | MA1:F4TCNQ (96%:4%) 20nm | MA1 150nm | MA2 70nm | IC1:TEG1 (90%:10%) 30nm | ST1:LiQ (50%:50%) 30nm | LiQ 1nm |
| E5* | MA2:F4TCNQ (96%:4%) 10nm | --- | MA2 170nm | M1:D2 (95%:5%) 20nm | ST1:LiQ (50%:50%) 30nm | LiQ 1nm |
| E6* | MA2:F4TCNQ (96%:4%) 10nm | --- | MA2 170nm | M1:D2 (95%:5%) 20nm | ST1 30nm | LiQ 3nm |
| E7* | MA1:F4TCNQ (96%:4%) 170nm | --- | MA1 10nm | M1:D2 (95%:5%) 20nm | ST1:LiQ (50%:50%) 30nm | LiQ 1nm |
| E8* | MA2:F4TCNQ (96%:4%) 170nm | --- | MA2 10nm | M1:D2 (95%:5%) 20nm | ST1:LiQ (50%:50%) 30nm | LiQ 1nm |
| E9* | MA2:F4TCNQ (97%:3%) 140nm | --- | MA2 20nm | M1:D2 (95%:5%) 20nm | ST1:LiQ (50%:50%) 30nm | - |
| E10* | MA1:F4TCNQ (96%:4%) 170nm | --- | MA1 10nm | M1:D2 (95%:5%) 20nm | ST1 30nm | LiQ 3nm |
| E11* | MA2:F4TCNQ (96%:4%) 170nm | --- | MA2 10nm | M1:D2 (95%:5%) 20nm | ST1 30nm | LiQ 3nm |
| E12 | MA1:F4TCNQ (96%:4%) 20nm | MA1 140nm | CbzA1 20nm | M1:D1 (95%:5%) 20nm | ST1:LiQ (50%:50%) 30nm | LiQ 1nm |
| E13 | MA1:F4TCNQ (96%:4%) 20nm | MA1 150nm | CbzA1 70nm | IC1:TEG1 (90%:10%) 30nm | ST1:LiQ (50%:50%) 40nm | LiQ 1nm |
| E14* | SpA1:F4TCNQ (96%:4%) 150nm | MA2:F4TCNQ (95%:5%) 20nm | MA2 10nm | M1:D2 (95%:5%) 20nm | ST1:LiQ (50%:50%) 30nm | LiQ 1nm |
| E15* | MA2:F4TCNQ (96%:4%) 20nm | --- | MA2 40nm | IC1:TEG1 (90%:10%) 30nm | ST1:LiQ (50%:50%) 40nm | --- |
| E16* | MA2:F4TCNQ (96%:4%) 20nm | --- | MA2 40nm | IC1:Cbz1:TEG1 (60%:30%:10%) 30nm | IC1 10nm ST1:LiQ (50%:50%) 30nm | --- |
| E17* | MA2:F4TCNQ (96%:4%) 20nm | --- | MA2 195nm | M1:D1 (95%:5%) 20nm | ST1:LiQ (50%:50%) 30nm | LiQ 1nm |
| E18 | MA2:F4TCNQ (96%:4%) 20nm | MA2 175nm | MA21 20nm | M1:D1 (95%:5%) 20nm | ST1:LiQ (50%:50%) 30nm | LiQ 1nm |
| E19 | MA2:F4TCNQ (96%:4%) 20nm | MA2 175nm | MA20 20nm | M1:D1 (95%:5%) 20nm | ST1:LiQ (50%:50%) 30nm | LiQ 1nm |

(fortgesetzt)

| Bsp | HTL1 Dicke | HTL2 Dicke | HTL3 Dicke | EML Dicke | ETL Dicke | EIL Dicke |
|---|---|---|---|---|---|---|
| E20 | MA2:F4TCNQ (96%:4%) 20nm | MA2 175nm | MA19 20nm | M1:D1 (95%:5%) 20nm | ST1:LiQ (50%:50%) 30nm | LiQ 1nm |
| E21 | MA2:F4TCNQ (96%:4%) 20nm | MA2 175nm | MA18 20nm | M1:D1 (95%:5%) 20nm | ST1:LiQ (50%:50%) 30nm | LiQ 1nm |
| E22 | MA2:F4TCNQ (96%:4%) 20nm | MA2 175nm | MA17 20nm | M1:D1 (95%:5%) 20nm | ST1:LiQ (50%:50%) 30nm | LiQ 1nm |
| E23 | MA2:F4TCNQ (96%:4%) 20nm | MA2 175nm | MA16 20nm | M1:D1 (95%:5%) 20nm | ST1:LiQ (50%:50%) 30nm | LiQ 1nm |
| E24 | MA2:F4TCNQ (96%:4%) 20nm | MA2 175nm | MA15 20nm | M1:D1 (95%:5%) 20nm | ST1:LiQ (50%:50%) 30nm | LiQ 1nm |
| E25 | MA2:F4TCNQ (96%:4%) 20nm | MA2 175nm | MA14 20nm | M1:D1 (95%:5%) 20nm | ST1:LiQ (50%:50%) 30nm | LiQ 1nm |
| E26 | MA2:F4TCNQ (96%:4%) 20nm | MA2 175nm | MA13 20nm | M1:D1 (95%:5%) 20nm | ST1:LiQ (50%:50%) 30nm | LiQ 1nm |
| E27 | MA2:F4TCNQ (96%:4%) 20nm | MA2 175nm | MA12 20nm | M1:D1 (95%:5%) 20nm | ST1:LiQ (50%:50%) 30nm | LiQ 1nm |
| E28 | MA2:F4TCNQ (96%:4%) 20nm | MA2 175nm | MA11 20nm | M1:D1 (95%:5%) 20nm | ST1:LiQ (50%:50%) 30nm | LiQ 1nm |
| E29 | MA2:F4TCNQ (96%:4%) 20nm | MA2 175nm | MA10 20nm | M1:D1 (95%:5%) 20nm | ST1:LiQ (50%:50%) 30nm | LiQ 1nm |
| E30 | MA2:F4TCNQ (96%:4%) 20nm | MA2 175nm | MA9 20nm | M1:D1 (95%:5%) 20nm | ST1:LiQ (50%:50%) 30nm | LiQ 1nm |
| E31 | MA2:F4TCNQ (96%:4%) 20nm | MA2 175nm | MA8 20nm | M1:D1 (95%:5%) 20nm | ST1:LiQ (50%:50%) 30nm | LiQ 1nm |
| E32 | MA2:F4TCNQ (96%:4%) 20nm | MA2 175nm | MA7 20nm | M1:D1 (95%:5%) 20nm | ST1:LiQ (50%:50%) 30nm | LiQ 1nm |
| E33 | MA2:F4TCNQ (96%:4%) 20nm | MA2 175nm | MA6 20nm | M1:D1 (95%:5%) 20nm | ST1:LiQ (50%:50%) 30nm | LiQ 1nm |
| E34 | MA2:F4TCNQ (96%:4%) 20nm | MA2 175nm | MA5 20nm | M1:D1 (95%:5%) 20nm | ST1:LiQ (50%:50%) 30nm | LiQ 1nm |
| E35 | MA2:F4TCNQ (96%:4%) 20nm | MA2 175nm | MA4 20nm | M1:D1 (95%:5%) 20nm | ST1:LiQ (50%:50%) 30nm | LiQ 1nm |
| E36 | MA2:F4TCNQ (96%:4%) 20nm | MA2 175nm | MA3 20nm | M1:D1 (95%:5%) 20nm | ST1:LiQ (50%:50%) 30nm | LiQ 1nm |
| E37 | MA2:F4TCNQ (96%:4%) 5nm | MA2 165nm | MA24 10nm | M1:D1 (95%:5%) 20nm | ST1:LiQ (50%:50%) 30nm | LiQ 1nm |
| E38 | MA2:F4TCNQ (96%:4%) 5nm | MA25 165nm | MA24 10nm | M1:D1 (95%:5%) 20nm | ST1:LiQ (50%:50%) 30nm | LiQ 1nm |
| E39 | MA2:F4TCNQ (96%:4%) 5nm | MA26 165nm | MA24 10nm | M1:D1 (95%:5%) 20nm | ST1:LiQ (50%:50%) 30nm | LiQ 1nm |
| E40 | MA2:F4TCNQ (96%:4%) 5nm | MA27 165nm | MA24 10nm | M1:D1 (95%:5%) 20nm | ST1:LiQ (50%:50%) 30nm | LiQ 1nm |

(fortgesetzt)

| Bsp | HTL1 Dicke | HTL2 Dicke | HTL3 Dicke | EML Dicke | ETL Dicke | EIL Dicke |
|---|---|---|---|---|---|---|
| E41 | MA9:F4TCNQ (94%:6%) 20nm | MA9 190nm | MA24 10nm | M1:D1 (95%:5%) 20nm | ST1:LiQ (50%:50%) 30nm | LiQ 1nm |
| E42 | MA9:F4TCNQ (94%:6%) 20nm | MA9 190nm | MA24 40nm | IC2:TEG1 (90%:10%) 30nm | IC2 10nm ST1:LiQ (50%:50%) 30nm | LiQ 1nm |
| E43 | MA29:F4TCNQ (94%:6%) 20nm | MA29 190nm | MA24 40nm | IC2:TEG1 (90%:10%) 30nm | IC2 10nm ST1:LiQ (50%:50%) 30nm | LiQ 1nm |
| E44 | MA2:F4TCNQ (96%:4%) 5nm | MA2 240nm | MA24 20nm | IC3:TEG2:TEY1 (84.6%:15%:0.4%) 20nm | IC1 10nm ST1:LiQ (50%:50%) 50nm | LiQ 1nm |
| E45 | MA28:F4TCNQ (94%:6%) 20nm | MA28 190nm | MA9 10nm | M1:D1 (95%:5%) 20nm | ST1:LiQ (50%:50%) 30nm | LiQ 1nm |
| E46* | MA28:F4TCNQ (94%:6%) 20nm | --- | MA9 200nm | M1:D1 (95%:5%) 20nm | ST1:LiQ (50%:50%) 30nm | LiQ 1nm |

Tabelle 3: Daten der OLEDs

| Bsp. | U1000 (V) | SE1000 (cd/A) | LE1000 (lm/W) | EQE 1000 | CIE x/y bei 1000 cd/2 | $j_{LD}$ | A | LD (h) |
|---|---|---|---|---|---|---|---|---|
| V1 | 4.4 | 7.3 | 5.2 | 5.2% | 0.14/0.19 | 80mA/cm$^2$ | 80% | 135 |
| V2 | 4.8 | 7.2 | 4.7 | 4.8% | 0.14/0.20 | 80 mA/cm$^2$ | 70% | 220 |
| V3 | 4.1 | 9.7 | 7.4 | 6.9% | 0.13/0.19 | 80 mA/cm$^2$ | 70% | 260 |
| V4 | 3.8 | 10.0 | 8.4 | 6.8% | 0.13/0.20 | 80 mA/cm$^2$ | 70% | 195 |
| V5 | 4.9 | 7.2 | 4.6 | 5.0% | 0.14/0.19 | 80 mA/cm$^2$ | 70% | 100 |
| V6 | 3.7 | 8.4 | 7.1 | 5.8% | 0.14/0.19 | 80 mA/cm$^2$ | 70% | 315 |
| V7 | 3.4 | 8.3 | 7.7 | 5.8% | 0.14/0.19 | 80 mA/cm$^2$ | 80% | 85 |
| V8 | 4.4 | 7.2 | 5.2 | 6.6% | 0.14/0.13 | 60 mA/cm$^2$ | 70% | 230 |
| V9 | 3.3 | 64 | 59 | 17.3% | 0.34/0.63 | 20 mA/cm$^2$ | 70% | 145 |
| V10 | 4.9 | 7.1 | 4.5 | 4.9% | 0.14/0.19 | 80 mA/cm$^2$ | 70% | 95 |
| V11 | 4.3 | 8.0 | 5.9 | 7.4% | 0.14/0.14 | 60 mA/cm$^2$ | 70% | 145 |
| V12 | 4.5 | 8.7 | 6.1 | 8.1% | 0.14/0.14 | 60mA/cm$^2$ | 70% | 100 |
| V13 | 4.4 | 8.7 | 6.3 | 8.1% | 0.14/0.13 | 60mA/cm$^2$ | 70% | 160 |
| V14 | 5.0 | 7.9 | 4.8 | 7.3% | 0.14/0.14 | 60mA/cm$^2$ | 70% | 105 |
| V15 | 4.8 | 9.2 | 6.0 | 8.5% | 0.14/0.14 | 60mA/cm$^2$ | 70% | 160 |
| V16 | 4.7 | 8.7 | 5.7 | 8.0% | 0.14/0.13 | 60mA/cm$^2$ | 70% | 215 |
| E1* | 4.0 | 8.2 | 6.4 | 7.4% | 0.14/0.13 | 60 mA/cm$^2$ | 70% | 460 |
| E2* | 3.9 | 8.7 | 7.0 | 8.0% | 0.14/0.13 | 60 mA/cm$^2$ | 70% | 435 |
| E3* | 3.0 | 78 | 82 | 21.1% | 0.34/0.63 | 20 mA/cm$^2$ | 70% | 215 |
| E4 | 3.2 | 70 | 70 | 19.4% | 0.33/0.63 | 20 mA/cm$^2$ | 70% | 205 |
| E5* | 4.1 | 10.4 | 7.9 | 7.3% | 0.13/0.19 | 80 mA/cm$^2$ | 70% | 435 |
| E6* | 3.5 | 10.2 | 9.2 | 7.2% | 0.14/0.19 | 80 mA/cm$^2$ | 70% | 280 |
| E7* | 3.7 | 9.0 | 7.7 | 5.9% | 0.14/0.20 | 80 mA/cm$^2$ | 70% | 425 |

(fortgesetzt)

| Bsp. | U1000 (V) | SE1000 (cd/A) | LE1000 (lm/W) | EQE 1000 | CIE x/y bei 1000 cd/$^2$ | $j_{LD}$ | A | LD (h) |
|---|---|---|---|---|---|---|---|---|
| E8* | 3.9 | 8.7 | 7.0 | 6.2% | 0.14/0.19 | 80 mA/cm$^2$ | 70% | 520 |
| E9* | 4.2 | 10.1 | 7.6 | 7.2% | 0.14/0.19 | 80 mA/cm$^2$ | 80% | 260 |
| E10* | 3.5 | 9.0 | 8.0 | 6.3% | 0.13/0.20 | 80 mA/cm$^2$ | 80% | 140 |
| E11* | 3.4 | 8.9 | 8.3 | 6.0% | 0.14/0.20 | 80 mA/cm$^2$ | 80% | 105 |
| E12 | 4.1 | 8.0 | 6.3 | 7.3% | 0.14/0.13 | 60 mA/cm$^2$ | 70% | 310 |
| E13 | 3.1 | 65 | 65 | 17.7% | 0.34/0.63 | 20 mA/cm$^2$ | 70% | 190 |
| E14* | 3.7 | 7.0 | 8.2 | 5.6% | 0.14/0.19 | 80mA/cm$^2$ | 70% | 430 |
| E15* | 3.1 | 76 | 78 | 20.7% | 0.34/0.62 | 20mA/cm$^2$ | 70% | 295 |
| E16* | 3.3 | 69 | 67 | 18.9% | 0.35/0.61 | 20mA/cm$^2$ | 80% | 450 |
| E17* | 4.0 | 8.3 | 6.6 | 7.9% | 0.14/0.13 | 60mA/cm$^2$ | 70% | 440 |
| E18 | 4.0 | 8.5 | 6.6 | 8.1% | 0.14/0.13 | 60mA/cm$^2$ | 70% | 450 |
| E19 | 4.1 | 8.1 | 6.2 | 7.7% | 0.14/0.13 | 60mA/cm$^2$ | 70% | 455 |
| E20 | 4.2 | 9.7 | 7.3 | 9.2% | 0.14/0.13 | 60mA/cm$^2$ | 70% | 540 |
| E21 | 4.0 | 7.7 | 6.0 | 7.3% | 0.14/0.13 | 60mA/cm$^2$ | 70% | 490 |
| E22 | 4.4 | 8.1 | 5.8 | 7.7% | 0.14/0.13 | 60mA/cm$^2$ | 70% | 525 |
| E23 | 4.1 | 9.0 | 7.0 | 8.6% | 0.14/0.12 | 60mA/cm$^2$ | 70% | 350 |
| E24 | 4.1 | 8.7 | 6.7 | 8.3% | 0.14/0.12 | 60mA/cm$^2$ | 70% | 485 |
| E25 | 4.0 | 8.3 | 6.6 | 7.9% | 0.14/0.12 | 60mA/cm$^2$ | 70% | 440 |
| E26 | 4.2 | 9.8 | 7.4 | 9.3% | 0.14/0.13 | 60mA/cm$^2$ | 70% | 355 |
| E27 | 3.9 | 9.5 | 7.6 | 9.1% | 0.14/0.13 | 60mA/cm$^2$ | 70% | 430 |
| E28 | 4.2 | 9.5 | 7.1 | 9.0% | 0.14/0.12 | 60mA/cm$^2$ | 70% | 315 |
| E29 | 4.0 | 9.9 | 7.8 | 9.4% | 0.14/0.12 | 60mA/cm$^2$ | 70% | 495 |
| E30 | 4.3 | 9.5 | 6.9 | 9.0% | 0.14/0.12 | 60mA/cm$^2$ | 70% | 430 |
| E31 | 4.5 | 10.1 | 7.1 | 9.6% | 0.14/0.12 | 60mA/cm$^2$ | 70% | 290 |
| E32 | 4.0 | 9.0 | 7.0 | 8.6% | 0.14/0.12 | 60mA/cm$^2$ | 70% | 450 |
| E33 | 4.3 | 9.1 | 6.7 | 8.7% | 0.14/0.14 | 60mA/cm$^2$ | 70% | 320 |
| E34 | 4.2 | 9.0 | 6.6 | 8.5% | 0.14/0.12 | 60mA/cm$^2$ | 70% | 445 |
| E35 | 4.4 | 9.3 | 6.7 | 8.8% | 0.14/0.13 | 60mA/cm$^2$ | 70% | 440 |
| E36 | 4.0 | 9.9 | 7.9 | 9.4% | 0.14/0.12 | 60mA/cm$^2$ | 70% | 330 |
| E37 | 4.0 | 9.6 | 7.6 | 9.1% | 0.14/0.13 | 50mA/cm$^2$ | 80% | 195 |
| E38 | 3.9 | 8.9 | 7.1 | 8.3% | 0.14/0.13 | 50mA/cm$^2$ | 80% | 180 |
| E39 | 4.0 | 8.1 | 6.4 | 7.5% | 0.14/0.13 | 50mA/cm$^2$ | 80% | 185 |
| E40 | 3.9 | 9.6 | 7.8 | 9.0% | 0.14/0.13 | 50mA/cm$^2$ | 80% | 320 |
| E41 | 3.9 | 9.2 | 7.3 | 8.0% | 0.13/0.15 | 50mA/cm$^2$ | 80% | 355 |
| E42 | 3.2 | 71 | 71 | 19.0% | 0.35/0.62 | 20mA/cm$^2$ | 80% | 215 |
| E43 | 2.9 | 69 | 75 | 18.9% | 0.33/0.63 | 20mA/cm$^2$ | 80% | 220 |
| E44 | 4.1 | 51 | 39 | 22.0% | 0.53/0.46 | 20mA/cm$^2$ | 80% | 670 |
| E45 | 3.9 | 9.2 | 7.3 | 8.0% | 0.14/0.15 | 50mA/cm$^2$ | 80% | 370 |

(fortgesetzt)

| Bsp. | U1000 (V) | SE1000 (cd/A) | LE1000 (lm/W) | EQE 1000 | CIE x/y bei 1000 cd/[2] | $j_{LD}$ | A | LD (h) |
|---|---|---|---|---|---|---|---|---|
| E46* | 3.9 | 9.1 | 7.3 | 8.0% | 0.14/0.14 | 50mA/$cm^2$ | 80% | 360 |

Tabelle 4: Strukturformeln der Materialien für die OLEDs

| | |
|---|---|
| 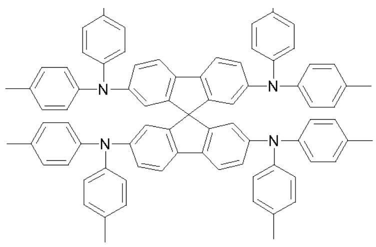 | 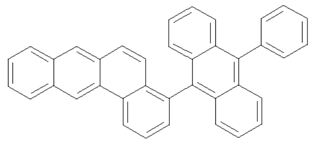 |
| SpA1 | M1 |
| 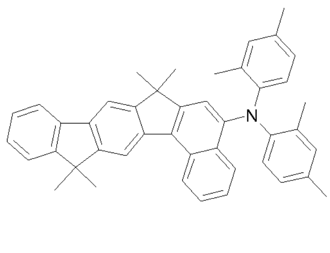 | 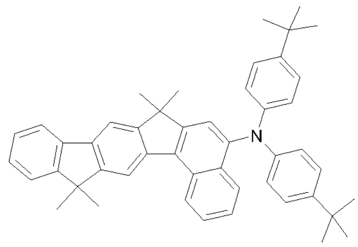 |
| D1 | D2 |
| 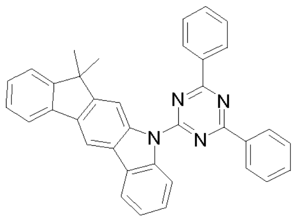 | 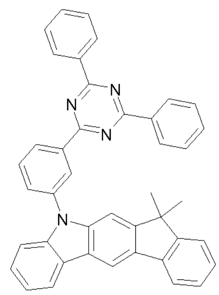 |
| IC1 | IC2 |
| 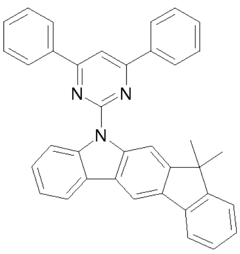 | |
| IC3 | |

(fortgesetzt)

| | |
|---|---|
| 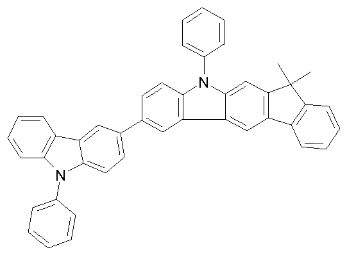 | 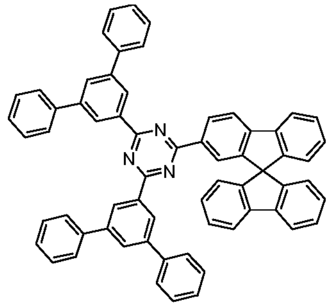 |
| Cbz1 | ST1 |
| 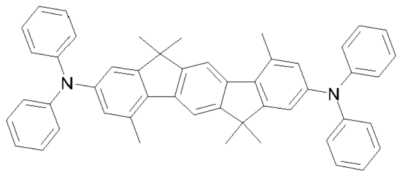 | 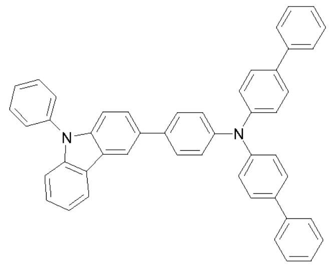 |
| TIFA1 | CbzA1 |
| 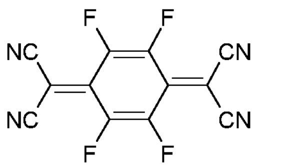 | 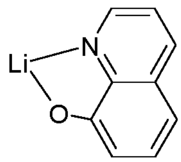 |
| F4TCNQ | LiQ |
| 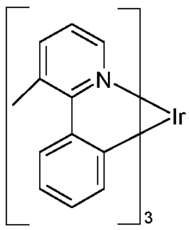 | 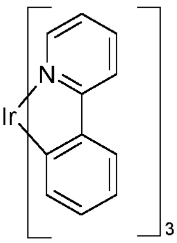 |
| TEG1 | TEG2 |
| 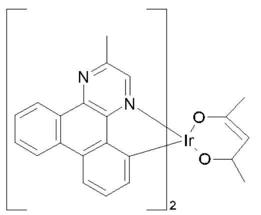 | 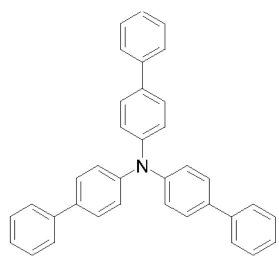 |
| TEY1 | MA1 |

(fortgesetzt)

| | |
|---|---|
| 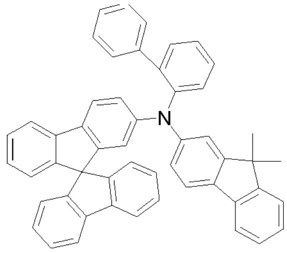 | 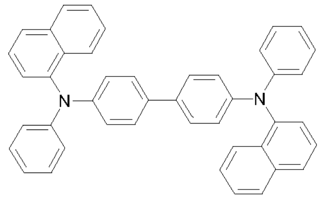 |
| MA2 | NPB |
| 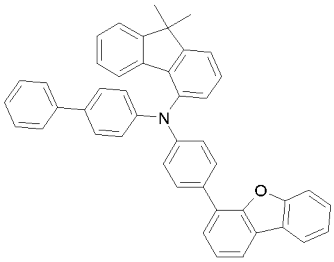 | 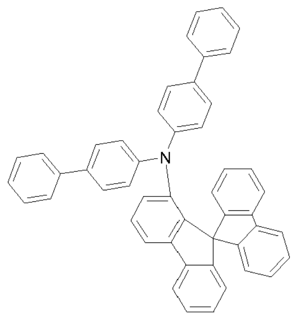 |
| MA3 | MA4 |
| 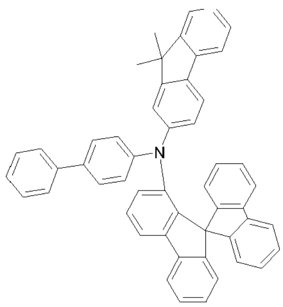 | 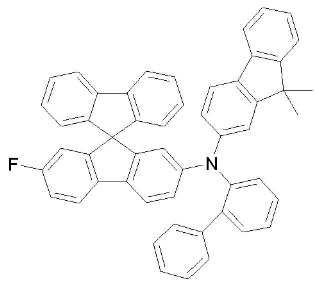 |
| MA5 | MA6 |
| 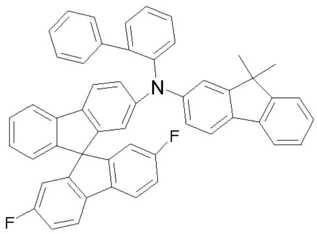 | 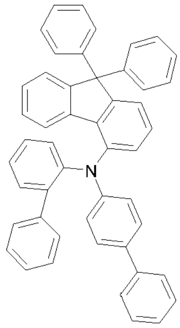 |
| MA7 | MA8 |

(fortgesetzt)

| | |
|---|---|
| 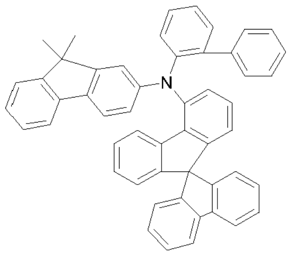 | 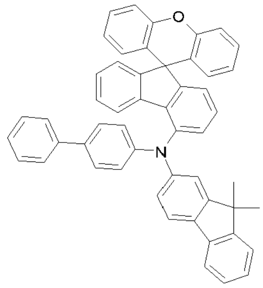 |
| MA9 | MA10 |
| 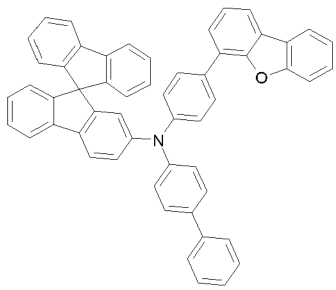 | 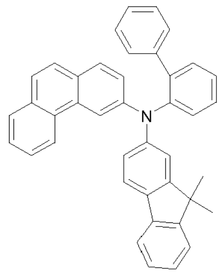 |
| MA11 | MA12 |
| 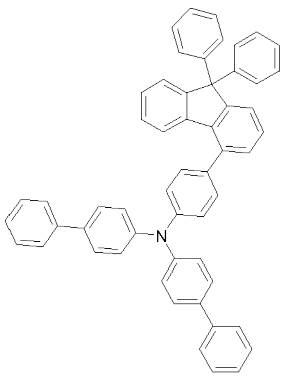 | 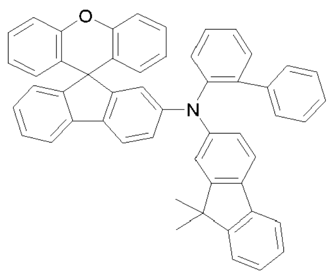 |
| MA13 | MA14 |
| 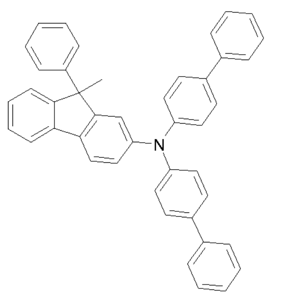 | 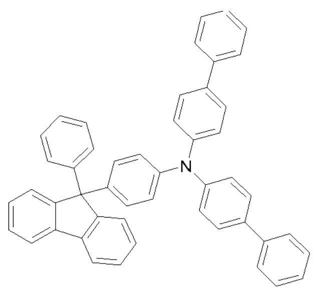 |
| MA15 | MA16 |

(fortgesetzt)

| | |
|---|---|
| 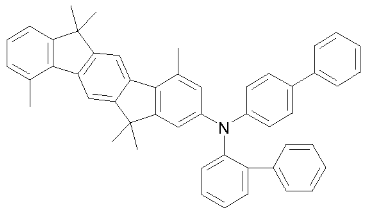 | 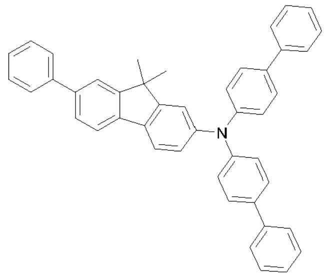 |
| MA17 | MA18 |
| 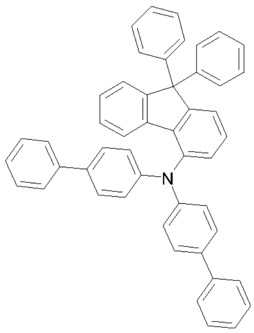 | 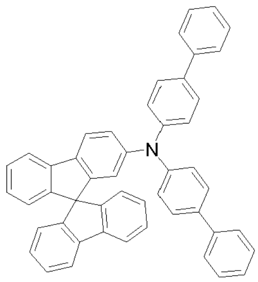 |
| MA19 | MA20 |
| 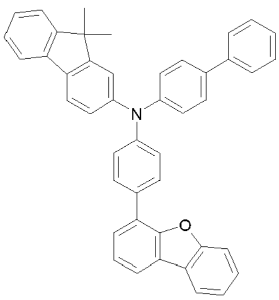 | 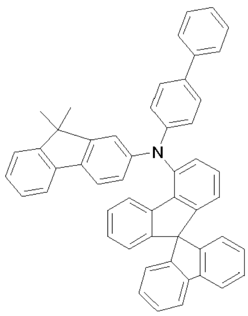 |
| MA21 | MA24 |
| 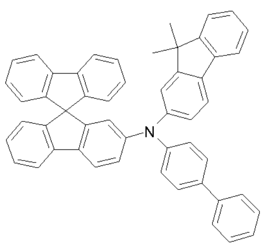 | 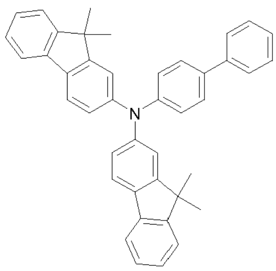 |
| MA25 | MA26 |

(fortgesetzt)

| | |
|---|---|
| 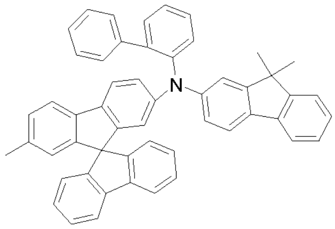 | 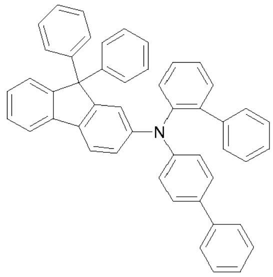 |
| MA27 | MA28 |
| 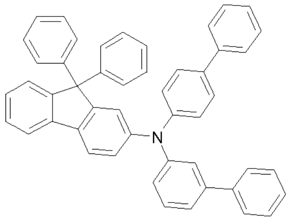 | |
| MA29 | |

## Patentansprüche

**1.** Elektronische Vorrichtung enthaltend

- Anode,
- Kathode,
- mindestens eine emittierende Schicht, welche zwischen Anode und Kathode angeordnet ist und je eine oder mehrere Emitterverbindungen enthält, die phosphoreszierend oder fluoreszierend sein können,
- mindestens eine p-dotierte Schicht A, welche zwischen Anode und emittierender Schicht angeordnet ist, und die ein Mono-Triarylamin als Host enthält, sowie
- mindestens eine nicht p-dotierte Schicht B, welche zwischen Anode und emittierender Schicht angeordnet ist, enthaltend ein Mono-Triarylamin, und
- eine weitere nicht p-dotierte Schicht C, enthaltend ein Mono-Triarylamin, zwischen der Schicht B und der emittierenden Schicht,

**dadurch gekennzeichnet, dass** die p-dotierte Schicht A direkt angrenzend an die Anode angeordnet ist, und dass die Schicht C unmittelbar an die emittierende Schicht angrenzt.

**2.** Elektronische Vorrichtung nach Anspruch 1, **dadurch gekennzeichnet, dass** ein oder mehrere Mono-Triarylamine identisch in allen Schichten zwischen Anode und emittierender Schicht vorhanden sind.

**3.** Elektronische Vorrichtung nach Anspruch 1 oder 2, **dadurch gekennzeichnet, dass** die p-dotierte Schicht A einen Dotanden enthält, der eine Elektronenakzeptorverbindung ist.

**4.** Elektronische Vorrichtung nach einem oder mehreren der Ansprüche 1 bis 3, **dadurch gekennzeichnet, dass** der Dotand der Schicht A gewählt ist aus Verbindungen, deren LUMO nicht höher liegt als 0.3 eV über dem HOMO des Mono-Triarylamins der Schicht A.

**5.** Elektronische Vorrichtung nach einem oder mehreren der Ansprüche 1 bis 4, **dadurch gekennzeichnet, dass** der Dotand der Schicht A gewählt ist aus Chinodimethanverbindungen, Azaindenofluorendionen, Azaphenalenen, Azatriphenylenen, $I_2$, Metallhalogeniden, Metalloxiden, und Übergangsmetallkomplexen.

**6.** Elektronische Vorrichtung nach einem oder mehreren der Ansprüche 1 bis 5, **dadurch gekennzeichnet, dass** der Dotand der Schicht A in einer Konzentration von 0.1 bis 20 Vol-% vorhanden ist.

7. Elektronische Vorrichtung nach einem oder mehreren der Ansprüche 1 bis 6, **dadurch gekennzeichnet, dass** das Mono-Triarylamin eine Verbindung der Formel (I) ist

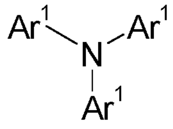

Formel (I)

wobei gilt:

$Ar^1$ ist bei jedem Auftreten gleich oder verschieden ein aromatisches oder heteroaromatisches Ringsystem mit 5 bis 60 aromatischen Ringatomen, welches durch einen oder mehrere Reste $R^1$ substituiert sein kann; und

$R^1$ ist bei jedem Auftreten gleich oder verschieden H, D, F, Cl, Br, I, CHO, $C(=O)R^2$, $P(=O)(R^2)_2$, $S(=O)R^2$, $S(=O)_2R^2$, $CR^2=CR^2R^2$, CN, $NO_2$, $Si(R^2)_3$, $OSO_2R^2$, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten $R^2$ substituiert sein kann, wobei eine oder mehrere nicht benachbarte $CH_2$-Gruppen durch $R^2C=CR^2$, $C\equiv C$ , $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S oder $CONR^2$ ersetzt sein können und wobei ein oder mehrere H-Atome durch D, F, Cl, Br, I, CN oder $NO_2$ ersetzt sein können, oder ein aromatisches oder hetero-aromatisches Ringsystem mit 5 bis 60 aromatischen Ring-atomen, das jeweils durch einen oder mehrere Reste $R^2$ substituiert sein kann, oder eine Aryloxy- oder Heteroaryloxy-gruppe mit 5 bis 60 aromatischen Ringatomen, die durch einen oder mehrere Reste $R^2$ substituiert sein kann, oder eine Kombination dieser Systeme; dabei können zwei oder mehrere benachbarte Substituenten $R^1$ auch miteinander ein mono- oder polycyclisches, aliphatisches oder aromatisches Ringsystem bilden;

$R^2$ ist bei jedem Auftreten gleich oder verschieden H, D, CN oder ein aliphatischer, aromatischer und/oder heteroaromatischer Kohlenwasserstoffrest mit 1 bis 20 C-Atomen, in dem auch H-Atome durch D oder F ersetzt sein können; dabei können zwei oder mehrere benachbarte Substituenten $R^2$ auch miteinander ein mono- oder polycyclisches, aliphatisches oder aromatisches Ringsystem bilden.

8. Elektronische Vorrichtung nach Anspruch 7, **dadurch gekennzeichnet, dass** mindestens eine der Gruppen $Ar^1$ ein aromatisches Ringsystem mit 12 bis 30 aromatischen Ringatomen darstellt, welches jeweils durch einen oder mehrere Reste $R^1$ substituiert sein kann.

9. Elektronische Vorrichtung nach einem oder mehreren der Ansprüche 1 bis 8, gewählt aus organischen integrierten Schaltungen (OICs), organischen Feld-Effekt-Transistoren (OFETs), organischen Dünnfilmtransistoren (OTFTs), organischen lichtemittierenden Transistoren (OLETs), organischen Solarzellen (OSCs), organischen optischen Detektoren, organischen Photorezeptoren, organischen Feld-Quench-Devices (OFQDs), organischen lichtemittierenden elektrochemischen Zellen (OLECs), organischen Laserdioden (O-Laser) und organischen Elektrolumineszenzvorrichtungen (OLEDs).

## Claims

1. Electronic device comprising

- anode,
- cathode,
- at least one emitting layer which is arranged between anode and cathode and in each case comprises one or more emitter compounds, which may be phosphorescent or fluorescent,
- at least one p-doped layer A which is arranged between anode and emitting layer and which comprises a monotriarylamine as host, as well as
- at least one non-p-doped layer layer B comprising a monotriarylamine which is arranged between anode and emitting layer and
- a further non-p-doped layer C, comprising a monotriarylamine, between layer B and the emitting layer,

**characterised in that** the p-doped layer A is arranged directly adjacent to the anode, and **in that** layer C is directly adjacent to the emitting layer.

2. Electronic device according to Claim 1, **characterised in that** one or more monotriarylamines are present identically in all layers between anode and emitting layer.

3. Electronic device according to Claim 1 or 2, **characterised in that** the p-doped layer A comprises a dopant which is an electron-acceptor compound.

4. Electronic device according to one or more of Claims 1 to 3, **characterised in that** the dopant of layer A is selected from compounds whose LUMO is not more than 0.3 eV higher than the HOMO of the monotriarylamine of layer A.

5. Electronic device according to one or more of Claims 1 to 4, **characterised in that** the dopant of layer A is selected from quinodimethane compounds, azaindenofluorenediones, azaphenalenes, azatriphenylenes, $I_2$, metal halides, metal oxides, and transition-metal complexes.

6. Electronic device according to one or more of Claims 1 to 5, **characterised in that** the dopant of layer A is present in a concentration of 0.1 to 20% by vol.

7. Electronic device according to one or more of Claims 1 to 6, **characterised in that** the monotriarylamine is a compound of the formula (I)

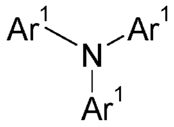

formula (I)

where:

$Ar^1$ is on each occurrence, identically or differently, an aromatic or heteroaromatic ring system having 5 to 60 aromatic ring atoms, which may be substituted by one or more radicals $R^1$;
$R^1$ is on each occurrence, identically or differently, H, D, F, Cl, Br, I, CHO, $C(=O)R^2$, $P(=O)(R^2)_2$, $S(=O)R^2$, $S(=O)_2R^2$, $CR^2=CR^2R^2$, CN, $NO_2$, $Si(R^2)_3$, $OSO_2R^2$, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thio-alkoxy group having 3 to 40 C atoms, which may in each case be substituted by one or more radicals $R^2$, where one or more non-adjacent $CH_2$ groups may be replaced by $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S or $CONR^2$ and where one or more H atoms may be replaced by D, F, Cl, Br, I, CN or $NO_2$, or an aromatic or heteroaromatic ring system having 5 to 60 aromatic ring atoms, which may in each case be substituted by one or more radicals $R^2$, or an aryloxy or heteroaryloxy group having 5 to 60 aromatic ring atoms, which may be substituted by one or more radicals $R^2$, or a combination of these systems; two or more adjacent substituents $R^1$ may also form a mono- or polycyclic, aliphatic or aromatic ring system with one another;
$R^2$ is on each occurrence, identically or differently, H, D, CN or an aliphatic, aromatic and/or heteroaromatic hydrocarbon radical having 1 to 20 C atoms, in which, in addition, H atoms may be replaced by D or F; two or more adjacent substituents $R^2$ may also form a mono- or polycyclic, aliphatic or aromatic ring system with one another.

8. Electronic device according to Claim 7, **characterised in that** at least one of the groups $Ar^1$ represents an aromatic ring system having 12 to 30 aromatic ring atoms, which may in each case be substituted by one or more radicals $R^1$.

9. Electronic device according to one or more of Claims 1 to 8, selected from organic integrated circuits (OICs), organic field-effect transistors (OFETs), organic thin-film transistors (OTFTs), organic light-emitting transistors (OLETs), organic solar cells (OSCs), organic optical detectors, organic photoreceptors, organic field-quench devices (OFQDs), organic light-emitting electrochemical cells (OLECs), organic laser diodes (O-lasers) and organic electroluminescent devices (OLEDs).

## Revendications

**1.** Dispositif électronique comprenant

- une anode,
- une cathode,
- au moins une couche d'émission qui est agencée entre l'anode et la cathode et qui, dans chaque cas, comprend un ou plusieurs composé(s) d'émetteur, laquelle peut être phosphorescente ou fluorescente,
- au moins une couche dopée selon le type p A qui est agencée entre l'anode et la couche d'émission et qui comprend une monotriarylamine en tant qu'hôte, de même que
- au moins une couche non dopée selon le type p B qui comprend une monotriarylamine, laquelle est agencée entre l'anode et la couche d'émission, et
- une autre couche non dopée selon le type p C, laquelle comprend une monotriarylamine, entre la couche B et la couche d'émission,

**caractérisé en ce que** la couche dopée selon le type p A est agencée de telle sorte qu'elle soit directement adjacente à l'anode, et **en ce que** la couche C est directement adjacente à la couche d'émission.

**2.** Dispositif électronique selon la revendication 1, **caractérisé en ce qu'**une ou plusieurs monotriarylamine(s) est/sont présente(s) de manière identique dans toutes les couches entre l'anode et la couche d'émission.

**3.** Dispositif électronique selon la revendication 1 ou 2, **caractérisé en ce que** la couche dopée selon le type p A comprend un dopant qui est un composé accepteur d'électrons.

**4.** Dispositif électronique selon une ou plusieurs des revendications 1 à 3, **caractérisé en ce que** le dopant de la couche A est sélectionné parmi les composés dont la LUMO n'est pas supérieure à la HOMO de la monotriarylamine de la couche A de plus de 0,3 eV.

**5.** Dispositif électronique selon une ou plusieurs des revendications 1 à 4, **caractérisé en ce que** le dopant de la couche A est sélectionné parmi les composés quinodiméthane, les azaindénofluorènediones, les azaphénalènes, les azatriphénylènes, $I_2$, les halogénures de métaux, les oxydes de métaux et les complexes de métaux de transition.

**6.** Dispositif électronique selon une ou plusieurs des revendications 1 à 5, **caractérisé en ce que** le dopant de la couche A est présent selon une concentration de 0,1 % à 20 % en volume.

**7.** Dispositif électronique selon une ou plusieurs des revendications 1 à 6, **caractérisé en ce que** la monotriarylamine est un composé de la formule (I)

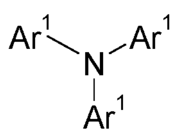

formule (I)

dans laquelle :

$Ar^1$ est pour chaque occurrence, de manière identique ou différente, un système de cycle aromatique ou hétéroaromatique qui comporte de 5 à 60 atomes de cycle aromatique, lequel peut être substitué par un radical ou par plusieurs radicaux $R^1$ ;
$R^1$ est pour chaque occurrence, de manière identique ou différente, H, D, F, Cl, Br, I, CHO, $C(=O)R^2$, $P(=O)(R^2)_2$, $S(=O)R^2$, $S(=O)_2R^2$, $CR^2=CR^2R^2$, CN, $NO_2$, $Si(R^2)_3$, $OSO_2R^2$, un groupe alkyle, alcoxy ou thioalcoxy en chaîne droite qui comporte de 1 à 40 atome(s) de C ou un groupe alkényle ou alkynyle en chaîne droite qui comporte de 2 à 40 atomes de C ou un groupe alkyle, alkényle, alkynyle, alcoxy ou thioalcoxy ramifié ou cyclique qui comporte de 3 à 40 atomes de C, lequel peut dans chaque cas être substitué par un radical ou par plusieurs radicaux $R^2$, où un ou plusieurs groupe(s) $CH_2$ non adjacents peut/peuvent être remplacé(s) par $R^2C=CR^2$, $C\equiv C$, $Si(R^2)_2$, $Ge(R^2)_2$, $Sn(R^2)_2$, C=O, C=S, C=Se, $C=NR^2$, $P(=O)(R^2)$, SO, $SO_2$, $NR^2$, O, S ou $CONR^2$ et où

un ou plusieurs atome(s) de H peut/peuvent être remplacé(s) par D, F, Cl, Br, I, CN ou $NO_2$, ou un système de cycle aromatique ou hétéroaromatique qui comporte de 5 à 60 atomes de cycle aromatique, lequel peut dans chaque cas être substitué par un radical ou par plusieurs radicaux $R^2$, ou un groupe aryloxy ou hétéroaryloxy qui comporte de 5 à 60 atomes de cycle aromatique, lequel peut être substitué par un radical ou par plusieurs radicaux $R^2$, ou une combinaison de ces systèmes ; deux substituants $R^1$ adjacents ou plus peuvent également former un système de cycle aliphatique ou aromatique monoou polycyclique l'un avec l'autre ou les uns avec les autres ;

$R^2$ est pour chaque occurrence, de manière identique ou différente, H, D, CN ou un radical hydrocarbone aliphatique, aromatique et/ou hétéroaromatique qui comporte de 1 à 20 atome(s) de C, où, en outre, des atomes de H peuvent être remplacés par D ou par F ; deux substituants $R^2$ adjacents ou plus peuvent également former un système de cycle aliphatique ou aromatique mono- ou polycyclique l'un avec l'autre ou les uns avec les autres.

8. Dispositif électronique selon la revendication 7, **caractérisé en ce qu'**au moins l'un des groupes $Ar^1$ représente un système de cycle aromatique qui comporte de 12 à 30 atomes de cycle aromatique, lequel peut dans chaque cas être substitué par un radical ou par plusieurs radicaux $R^1$.

9. Dispositif électronique selon une ou plusieurs des revendications 1 à 8, sélectionné parmi les circuits intégrés organiques (OIC), les transistors à effet de champ organiques (OFET), les transistors à film mince organiques (OTFT), les transistors à émission de lumière ou électroluminescents organiques (OLET), les cellules solaires organiques (OSC), les détecteurs optiques organiques, les photorécepteurs organiques, les dispositifs à extinction de champ organiques (OFQD), les cellules électrochimiques à émission de lumière ou électroluminescentes organiques (OLEC), les diodes laser organiques (O-laser) et les dispositifs électroluminescents organiques (OLED).

Fig. 1

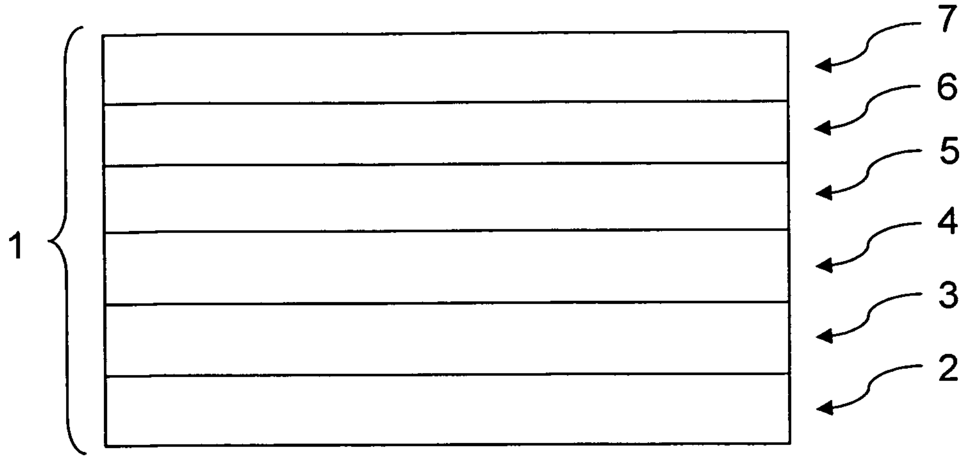


Fig. 2

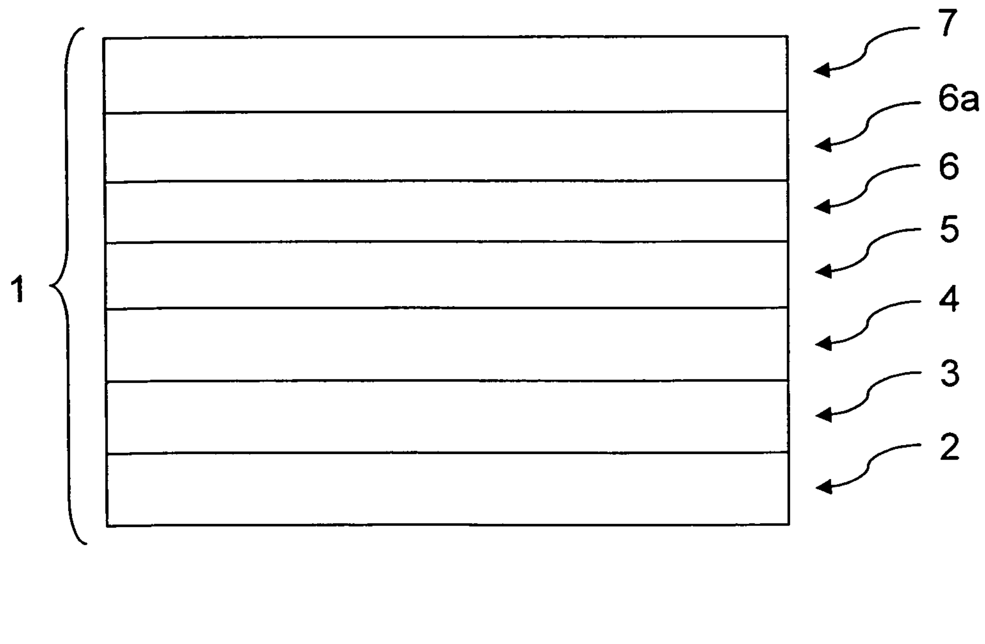

Fig. 3

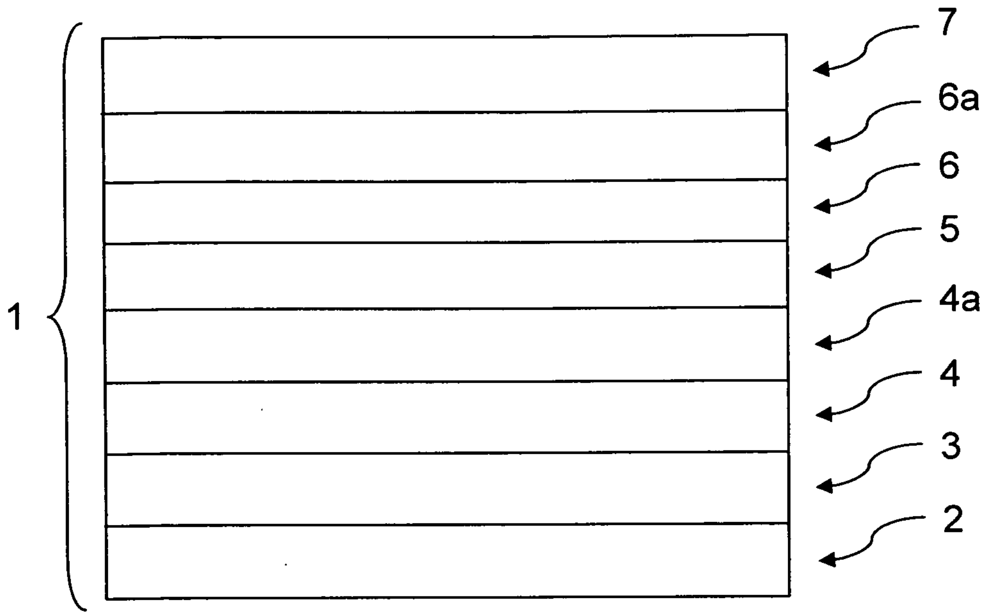


Fig. 4

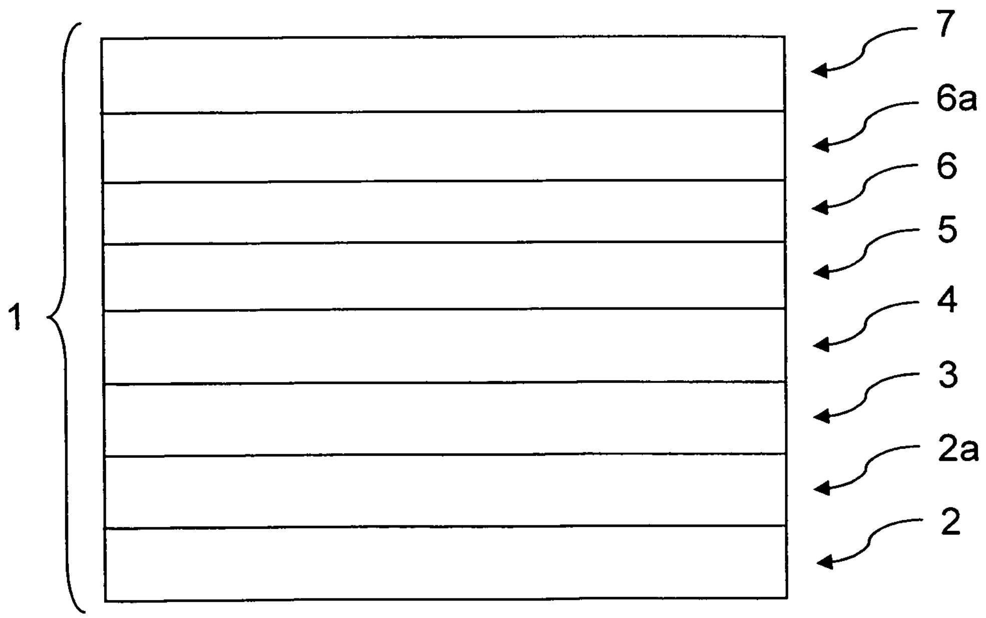

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1463130 A2
- DE 102007031220 A1
- US 5093698 A
- EP 1885008 A1
- JP 1995053955 A
- EP 2365555 A2
- WO 2011073149 A
- EP 1968131 A
- EP 2276085 A
- EP 2213662 A
- EP 1722602 A
- EP 2045848 A
- DE 102007031220
- US 8044390 B
- US 8057712 B
- WO 2009003455 A
- WO 2010094378 A
- WO 2011120709 A
- US 20100096600 A
- WO 2012095143 A
- EP 1786050 A1
- WO 2005011013 A
- WO 200070655 A
- WO 200141512 A
- WO 200202714 A
- WO 200215645 A
- EP 1191613 A
- EP 1191612 A
- EP 1191614 A
- WO 2005033244 A
- WO 2005019373 A
- US 20050258742 A
- EP 676461 A
- WO 2004081017 A
- WO 2004058911 A
- WO 2005084081 A
- WO 2005084082 A
- WO 2006048268 A
- WO 2006117052 A
- WO 2008145239 A
- US 20050069729 A
- WO 2005039246 A
- JP 2004288381 A
- EP 1205527 A
- WO 2008086851 A
- WO 2011088877 A
- WO 2011128017 A
- WO 2010136109 A
- WO 2011000455 A
- EP 1617710 A
- EP 1617711 A
- EP 1731584 A
- JP 2005347160 A
- WO 2007063754 A
- WO 2008056746 A
- WO 2004093207 A
- WO 2010006680 A
- WO 2005003253 A
- WO 2007137725 A
- WO 2005111172 A
- WO 2010015306 A
- EP 652273 A
- WO 2009062578 A
- WO 2010054729 A
- WO 2010054730 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- EP 12000929
- EP 12005369
- EP 12005370
- EP 12005371
- EP 11009127
- EP 11007067
- JP 7053955 A


### In der Beschreibung aufgeführte Nicht-Patentliteratur


- *Chem. Rev.,* 2007, vol. 107, 1233
- **PAUW et al.** *Philips Technical Review,* 1959, vol. 20, 220

- **PAUW et al.** *Philips Research Reports,* 1958, vol. 13, 1
- **Y. SHIROTA et al.** *Chem. Rev.,* 2007, vol. 107 (4), 953-1010
- *Solar Energy Materials & Solar Cells,* 2000, vol. 63, 83-99
- **B. M. S. ARNOLD et al.** *Appl. Phys. Lett.,* 2008, vol. 92, 053301